# EUROPEAN PATENT SPECIFICATION

(11) **EP 2 220 150 B1**
(45) Date of publication and mention of the grant of the patent: **06.11.2013**
(21) Application number: 08855728.5
(22) Date of filing: 26.11.2008
(51) Int. Cl.: B29C 55/00, C08J 9/26, C08J 9/28, C08J 9/00, A61K 47/30, C08K 3/34, C08K 5/00, C08K 5/11

(54) **METHODS FOR FORMING MICROPOROUS AND ANTIMICROBIAL ARTICLES**
VERFAHREN ZUR BILDUNG VON MIKROPORÖSEN UND ANTIMIRKOBIELLEN GEGENSTÄNDEN
PROCÉDÉS DE FORMATION D'ARTICLES MICROPOREUX ET ANTIMICROBIENS

(30) Priority: 26.11.2007 US 989999 P
(43) Date of publication of application: 25.08.2010
(73) Proprietor: 3M Innovative Properties Company, Saint Paul, MN 55133-3427 (US)
(72) Inventor: SAKURAI, Aizoh, Setagaya-ku Tokyo 158-8583 (JP); SAITO, Kana, Setagaya-ku Tokyo 158-8583 (JP); TORIUMI, Naoyuki, Setagaya-ku Tokyo 158-8583 (JP); MROZINSKI, James S., Saint Paul, Minnesota 55133-3427 (US); SCHMID, Matthew J., Saint Paul, Minnesota 55133-3427 (US)
(74) Representative: Vossius & Partner
(86) International application number: PCT/US2008/084774
(87) International publication number: WO 2009/070630

(56) References cited:
- WO-A-00/62829
- US-A1- 2004 265 565
- US-A1- 2005 058 821

## Description

### Field

The present disclosure relates to forming microporous and antimicrobial articles.

### Background

Microporous materials are used in a wide range of applications including fibers, breathable membranes, absorbent articles, filtration articles, and diffusion barriers or separators in electrochemical cells. Numerous methods have been used for making microporous materials.

Thermally induced phase separation methods have been used to prepare microporous materials. Thermally induced phase separation occurs when a polymer is soluble in a diluent at an elevated temperature and is insoluble in the same diluent at a lower temperature. The polymer and the diluent each separate into polymer rich and diluent rich regions at the lower temperature. Phase separation can occur by liquid-liquid mechanisms, solid-liquid mechanisms, or combinations of liquid-liquid and solid-liquid mechanisms. Phase separation mechanisms have been described in U.S. Patent Nos. 4,539,256 (Shipman); 4,247,498 (Castro); and 4,867,881 (Kinzer).

### Summary

The present disclosure describes methods for forming microporous and antimicrobial articles. More specifically, a method for forming a microporous article comprising crystalline or semicrystalline polylactic acid material is described. Semicrystalline or crystalline polylactic acid material, a nonpolymeric aliphatic ester diluent, and a nucleating agent are heated above the melting temperature of the crystalline or semicrystalline polylactic acid material to form a melt blended composition. The melt blended composition is then cooled such that the crystalline or semicrystalline polylactic acid material and the nonpolymeric aliphatic ester diluent phase separate into a composition having two continuous phases. A network of interconnected micropores is formed within the composition by stretching in at least one direction.

In a first aspect, a method is provided for forming a microporous article. The method includes preparing an initial composition comprising a crystalline or semicrystalline polylactic acid material, a nonpolymeric aliphatic ester diluent, and a nucleating agent. The method further includes heating the initial composition to at least the melting temperature of the crystalline or semicrystalline polylactic acid material to form a melt blended composition. The crystalline or semicrystalline polylactic acid material and the nonpolymeric aliphatic ester diluent of the melt blended composition form a single liquid phase. The nucleating agent of the melt blended composition is uniformly dispersed or dissolved in the single liquid phase. The melt blended composition is then cooled to a temperature sufficient for the melt blended composition to phase separate into a composition having two continuous phases. The composition comprises a first phase and a second phase. The first phase contains a crystalline or semicrystalline polylactic acid matrix and the nucleating agent dispersed throughout the crystalline or semicrystalline polylactic acid matrix. The second phase contains the nonpolymeric aliphatic ester diluent. A network of interconnected micropores is formed by stretching the composition in at least one direction, by a combination of stretching in at least one direction and removing at least a portion of the nonpolymeric aliphatic ester diluent before or after stretching.

In a second aspect, a composition obtainable by the above method before stretching having two continuous phases comprising a first phase and a second phase is provided. More specifically, the composition comprises a first phase having 40 to 80 weight percent of a crystalline or semicrystalline polylactic acid material, and 0.01 to 10 weight percent of a nucleating agent. The second phase comprises 20 to 60 weight percent of a nonpolymeric aliphatic ester diluent. The weight percent of the crystalline or semicrystalline polylactic acid material, the nucleating agent, and the nonpolymeric aliphatic ester diluent are each independently based on the total weight of the composition. The first phase is at least partially surrounded by the second phase.

In a third aspect, a microporous article obtainable by the above method, comprising a crystalline or semicrystalline polylactic acid material, a nucleating agent, and a nonpolymeric aliphatic ester diluent is provided. Some or all of the nonpolymeric aliphatic ester diluent may be removed from the microporous article. The microporous article has a network of interconnected micropores therebetween characterized by a multiplicity of spaced, spherulitic shaped crystalline or semicrystalline polylactic acid material domains. Adjacent crystalline or semicrystalline polylactic acid material domains are connected to each other by a plurality of fibrils comprising polylactic acid material.

In a fourth aspect, an antimicrobial microporous article obtainable by the above method comprising a crystalline or semicrystalline polylactic acid material, a nucleating agent, an antimicrobial component, an enhancer, and a nonpolymeric aliphatic ester diluent are provided. Some or all of the nonpolymeric aliphatic ester diluent may be removed from the microporous article. The antimicrobial microporous article has a network of interconnected micropores therebetween characterized by a multiplicity of spaced, spherulitic shaped crystalline or semicrystalline polylactic acid material domains. Adjacent crystalline or semicrystalline polylactic acid material domains are connected to each other by a plurality of fibrils comprising polylactic acid material.

### Brief Description of the Drawings

**FIG. 1** is a scanning electron micrograph (SEM) of a microporous article of Example 5 before stretching.
**FIG. 2** is a scanning electron micrograph (SEM) of a microporous article of Example 10 after stretching.

### Detailed Description

For the following defined terms, these definitions shall be applied, unless a different definition is given in the claims or elsewhere in the specification.

The term "Microporous" refers to films, membranes or film layers having average pore sizes in a range of 0.1 micrometers to 100 micrometers, 0.1 micrometers to 85 micrometers, 0.1 micrometers to 70 micrometers, 0.1 micrometers to 50 micrometers, 0.1 micrometers to 25 micrometers, 0.1 micrometers to 15 micrometers, or in a range of 0.1 micrometers to 10 micrometers. Average pore sizes can be described by bubble point measurements (ASTM-F-316-80).

The term "semicrystalline" refers to polymeric materials having structures which are at least partially crystalline. The polymeric materials generally have a crystallinity of greater than 10 weight percent as measured by Differential Scanning Calorimetry (DSC). The percent crystallinity can be determined by quantifying the heat associated with melting of the polymeric material with DSC. The heat can be reported as percent crystallinity by normalizing the observed heat of fusion to that of a 100 percent crystalline sample, if known, of the same polymeric material. For example, the crystallinity can be greater than 25 weight percent, greater than 30 weight percent, greater than 40 weight percent, or greater than 50 weight percent. The terms "crystalline" and "semi-crystalline" can be interchangeable.

The term "melting temperature" refers generally to a temperature at which a semicrystalline polymeric material transitions from a solid to a liquid. More specifically, a peak of a melting endotherm of the semicrystalline polymeric material as recorded by DSC can be described as the melting temperature. The breadth of the endotherm peak is primarily related to the size and degree of perfection of the polymer crystals of the polymeric material. An amorphous polymeric material typically lacks a melting endotherm when analyzed by DSC. Other analytical techniques may be used to determine the melting temperature of a semicrystalline polymeric material.

The term "solid-liquid phase separation" refers to a mechanism for separating the melt blended composition comprising at least a polymer and a diluent. The melt blended composition may further comprise a nucleating agent. As the melt blended composition is cooled, the nucleating agent may initiate the formation of crystallization sites within the polymer. The diluent phase separates from the crystallized sites of polymer resulting in the formation of two phases. The phase separated composition comprises a first phase having a crystalline or semicrystalline polymer and a second phase having a diluent. The first and second phases form a composition having two continuous phases.

The term "liquid-liquid phase separation" refers to a mechanism for separating the melt blend composition comprising at least a polymer and a diluent. The melt blended composition may further comprise a nucleating agent. The diluent phase may separate from the melted polymer resulting in the formation of two phases. The phase separated composition comprises a first phase having a diluent and a second phase comprising a polymer.

The term "combination of solid-liquid phase separation and liquid-liquid phase separation" refers to the combination of both phase separation mechanisms described above.

The term "miscible" refers particularly to various substances, particularly liquids (e.g., melted polymeric materials and diluents), capable of mixing in any ratio to form a single phase that will not separate into different phases. The substances form a homogeneous mixture and are considered to be soluble in all proportions with one another without phase separation.

The term "immiscible" refers particularly to various substances, particularly liquids (e.g., melted polymeric materials and diluents), that are incapable of being mixed or blended to form a single phase, but after even after mixing, separate into different phases.

The term "hydrophilic" refers to a material having a property of promoting the penetration of water. A hydrophilic surface of a material generally has a contact angle less than 90 degrees. The surface may be considered hydrophilic when water is absorbed into the material. Hydrophilic surfaces may absorb generally polar substances. For example, a microporous article may be classified as hydrophilic based on the amount of time needed for water to be absorbed through a surface or to change the transparency of an article.

The term "hydrophobic" refers to a material having a property of preventing or inhibiting penetration of water. A hydrophobic surface of a material generally has a contact angle greater than 90 degrees. A hydrophobic surface may absorb oil or generally nonpolar substances. For example, a microporous article may be classified as hydrophobic based on the amount of time needed for oil or a nonpolymeric aliphatic ester diluent to be absorbed through a surface or to change the transparency of an article.

The term "antimicrobial" or "antimicrobial activity," used as an adjective, means the ability to kill pathogenic and non-pathogenic microorganisms including bacteria, fungi, algae and virus. Preferred antimicrobial materials exhibit at least 1 log reduction, preferably 2 log reduction, and most preferably 4 log reduction of S. aureus (AATC 25923) in 60 minutes from an initial inoculum of 1-3 x 10⁷ cfu/ml when tested in Mueller Hinton broth at 35 °C at a concentration of 0.25 wt. % in a Rate of Kill assay using an appropriate neutralizer as described in G. Nicoletti, V. Boghossian, F. Gurevitch, R. Borland and P. Mogenroth, "The Antimicrobial Activity in Vitro of Chlorhexidine, a Mixture of Isothiazolinones (Kathon CG) and Cetyl Trimethyl Ammonium Bromide (CTAB"), Journal of Hospital Infection, vol. 23, pp. 87-111, (1993). The concentrations or amounts of the antimicrobial components, when considered separately, may not kill to an acceptable level, may not kill as broad a spectrum of undesired microorganisms, or may not kill as fast; however, when the antimicrobial and an enhance are provided, such components provide an enhanced antimicrobial activity (as compared to the same components used alone under the same conditions). Measurable antimicrobial activity is further described in American Association of Textile and Color Chemists (AATCC) Test Method 100-2004 (AATCC Technical manual, Vol. 80, 2005, pp. 149-151) and Japanese Industrial Standard (JIS) Z 2801:2000 (Japanese Standards Association, 2001, pp. 1-11).

The term "biodegradable" means degradable by the action of naturally occurring microorganisms such as bacteria, fungi and algae and/or natural environmental factors such as hydrolysis, transesterification, exposure to ultraviolet or visible light (photodegradable) and enzymatic mechanisms or combinations thereof.

The term "biocompatible" means biologically compatible by not producing toxic, injurious or immunological response in living tissue. Biocompatible materials may also be broken down by biochemical and/or hydrolytic processes and absorbed by living tissue. Test methods used include ASTM F719 for applications where the compositions contact tissue such as skin, wounds, mucosal tissue including in an orifice such as the esophagus or urethra, and ASTM F763 for applications where the compositions are implanted in tissue.

The term "sufficient amount" or "effective amount" means the amount of the antimicrobial component and/or enhancer when in a composition, as a whole, provides an antimicrobial (including, for example, antiviral, antibacterial, or antifungal) activity that reduces, prevents growth of, or eliminates colony forming units for one or more species of microorganisms such that an acceptable level of the organism results.

The term "enhancer" refers to a component that enhances the antimicrobial activity of an antimicrobial component. The enhancing effect can be with respect to the level of kill, the speed of kill, and/or the spectrum of microorganisms killed, and may not be seen for all microorganisms. In fact, an enhanced level of kill is most often seen in Gram negative bacteria such as *Escherichia coli.* An enhancer may be a synergist such that when combined with the remainder of the composition, the combined antimicrobial activity is greater than the sum of the activity of the composition without the enhancer component and the composition without the antimicrobial component.

The term "antimicrobial component" means an antiseptic that generally is a small molecule having a molecular weight less than about 1000 Daltons, and often less than 500 Daltons and that display antimicrobial activity in the presence of at least one species of bacteria, fungi, and/or virus. Preferred antimicrobial components are lipophilic preferably having a solubility in water of no greater than 1.0 gram per 100 grams (1.0g/100g) deionized water. For prolonged use applications, preferred antimicrobial components or antimicrobial lipids have a solubility in water of no greater than 0.5g/100g deionized water, more preferably, no greater than 0.25g/100g deionized water, and even more preferably, no greater than 0.10g/100g deionized water. Solubilities are described using radio-labeled compounds as described under "Conventional Solubility Estimations" in Solubility of Long-Chain Fatty Acids in Phosphate Buffer at ph 7.4, Henrik Vorum et.al., in Biochimica et. Biophysica Acta., 1126, 135-142(1992). Preferred antimicrobial components have a solubility in deionized water of at least 100 micrograms (µg) per 100 grams deionized water, more preferably, at least 500 µg/100g deionized water, and even more preferably, at least 1000 µg/100g deionized water.

The term "fatty" means a straight or branched chain alkyl or alkylene moiety having 6 to 22 (odd or even number) carbon atoms, unless otherwise specified.

The recitation of numerical ranges by endpoints includes all numbers subsumed within that range (e.g., 1 to 5 includes 1, 1.5, 2, 2.75, 3, 3.8, 4, and 5).

As included in this specification and the appended claims, the singular forms "a", "an", and "the" include plural referents unless the content clearly dictates otherwise. Thus, for example, reference to a composition containing "a compound" includes a mixture of two or more compounds. As used in this specification and appended claims, the term "or" is generally employed in its sense including "and/or" unless the content clearly dictates otherwise.

Unless otherwise indicated, all numbers expressing quantities or ingredients, measurement of properties and so forth used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings of the present disclosure. At the very least, each numerical parameter should at least be construed in light of the number of reported significant digits and by applying ordinary rounding techniques. Notwithstanding that the numerical ranges and parameters setting forth the broad scope of the disclosure are approximations, the numerical values set forth in the specific examples are reported as precisely as possible. Any numerical value, however, inherently contains errors necessarily resulting from the standard deviations found in their respective testing measurements.

In the inventive method, a melt blended composition is cooled sufficiently to a temperature at which the nucleating agent induces crystallization of the polylactic acid material from the single liquid phase. During crystallization of the polylactic acid material, the melt blended composition phase separates into a composition having two continuous phases that comprises a first phase and a second phase. The first phase comprises a crystallized polylactic acid material as a crystalline or semicrystalline polylactic acid matrix and the nucleating agent dispersed throughout the crystalline or semicrystalline polylactic acid matrix. The second phase comprises the nonpolymeric aliphatic ester diluent. Phase separation of the melt blended composition may comprise a solid-liquid phase separation mechanism, a liquid-liquid phase separation mechanism, or a combination of solid-liquid and liquid-liquid phase separation mechanisms.

The semicrystalline polylactic acid material is a solid at room temperature, such that the semicrystalline polylactic acid material melts when heated to at least its melting temperature. The nonpolymeric aliphatic ester diluent may be a liquid or a solid at room temperature. The nonpolymeric aliphatic ester diluent does not dissolve the semicrystalline polylactic acid material below the melting temperature of the semicrystalline polylactic acid material. The melting temperature of the semicrystalline polylactic acid material may decrease in the presence of the nonpolymeric aliphatic ester diluent. The nonpolymeric aliphatic ester diluent is selected to be miscible with the semicrystalline polylactic acid material at or above the melting temperature of the semicrystalline polylactic acid material. The nucleating agent can be a solid or a liquid. Further, the nucleating agent may disperse in the nonpolymeric aliphatic ester diluent prior to heating the initial composition.

Polymer blends containing biodegradable materials such as semicrystalline polymeric materials can be used for forming microporous articles. Semicrystalline polylactic acid material is one example of a biodegradable polymeric material useful for forming microporous articles. Semicrystalline polylactic acid materials used in the initial composition include polylactic acid materials that are generally known to those skilled in the art. Generally, semicrystalline polylactic acid materials are melt-processable. Upon heating, thermoplastic semicrystalline polylactic acid material will easily soften and/or melt to permit processing in conventional equipment for forming microporous articles. Upon cooling from temperatures at or above the melting temperature of the semicrystalline polylactic acid material, the polylactic acid material crystallizes in the presence of a nucleating agent to form geometrically regular and ordered chemical regions.

Semicrystalline polylactic acid materials are generally solid at room temperature and can not be dissolved with a nonpolymeric aliphatic ester diluent below the melting temperature of the semicrystalline polylactic acid material. The semicrystalline polylactic acid material generally does not dissolve in the nonpolymeric aliphatic ester diluent at room temperature. At a temperature at or above the crystallization temperature of the semicrystalline polylactic acid material, the nonpolymeric aliphatic ester diluent forms a single liquid phase with the semicrystalline polylactic acid material. Upon cooling the single liquid phase, the polylactic acid material crystallizes forming a first phase, and the nonpolymeric aliphatic ester diluent forms a second phase. The crystallization temperature of the semicrystalline polylactic acid material is a temperature from which the polymer chains tend to orient into ordered arrangements for forming crystalline regions within the polylactic acid material. The polylactic acid material begins to crystallize at or below the crystallization temperature. The first phase and the second phase form a composition.

Some representative semicrystalline polylactic acid materials that can be used to form microporous articles include, but are not limited to, a poly(L-lactic acid) material where the constituent units comprise only L-lactic acid; a poly(D-lactic acid) material where the constituent units comprise only D-lactic acid; and a poly (D/L-lactic acid) material where both L-lactic acid units and D-lactic acid units are present in various ratios. Generally, the poly(D/L-lactic acid) material has a high enantiomeric ratio (e.g., a high L-lactic acid:low D-lactic acid ratio or a high D-lactic acid:low L-lactic acid ratio) to maximize the intrinsic crystallinity of the polylactic acid material. The degree of crystallinity of a polylactic acid material is based on the stereoregularity of the polymer backbone, and the ability to crystallize with other polymer chains. Copolymers of L- or D-lactic acid with an aliphatic hydroxycarboxylic acid monomer other than lactic acid such as glycolic acid, 3-hydroxybutyric acid, 4-hydroxybutyric acid, 4-hydroxyvaleric acid, 5-hydroxyvaleric acid or 6-hydroxycaproic acid may be used to form a semicrystalline polylactic acid material. The percent of a comonomer, such as an aliphatic hydroxycarboxylic acid monomer, with lactic acid in a copolymer is kept in a range to provide a semicrystalline polylactic acid material. Semicrystalline polylactic acid materials as described may be used alone, or two or more different semicrystalline polylactic acid materials may be used in combination.

One example of a semicrystalline polylactic acid material may include an aliphatic polyester resin containing a lactic acid unit, an aliphatic polyvalent carboxylic acid unit and an aliphatic polyhydric alcohol unit. The aliphatic polyester resin may be formed from a combination of an aliphatic polyvalent carboxylic acid and an aliphatic polyhydric alcohol with a lactic acid unit such as polylactic acid, or a copolymer of lactic acid and another hydroxycarboxylic acid. Alternatively, the aliphatic polyester resin may be formed from a combination of a polyvalent carboxylic acid and an aliphatic polyhydric alcohol with lactic acid. The aliphatic polyester resin may be formed from a combination of an aliphatic polyvalent carboxylic acid and an aliphatic polyhydric alcohol with a lactide, or with a cyclic ester of a hydroxycarboxylic acid.

Specific examples of aliphatic polyvalent carboxylic acids that may be used for forming an aliphatic polyester resin include, but are not limited to, oxalic acid, succinic acid, malonic acid, glutaric acid, adipic acid, pimelic acid, suberic acid, azelaic acid, undecanoic diacid, dodecanoic diacid, and anhydrides thereof. Such aliphatic polyvalent carboxylic acids may be used as an acid anhydride or a mixture with the acid anhydride. Specific examples of the aliphatic polyhydric alcohol include, but are not limited to, ethylene glycol, diethylene glycol, triethylene glycol, propylene glycol, dipropylene glycol, 1,3-butanediol, 1,4-butanediol, 3-methyl-1,5-pentanediol, 1,6-hexanediol, 1,9-nonanediol, neopentyl glycol, tetramethylene glycol and 1,4-cyclohexane dimethanol.

Another example of a semicrystalline polylactic acid material may include an aliphatic polyester resin containing a lactic acid unit and a polyfunctional polysaccharide. The aliphatic polyester resin can be formed from a polyfunctional polysaccharide with a lactic acid unit such as polylactic acid or a copolymer of lactic acid and another hydroxycarboxylic acid. Alternatively, the aliphatic polyester resin may be formed from a polyfunctional polysaccharide with a lactide, or a cyclic ester of a hydroxycarboxylic acid. Specific examples of polyfunctional polysaccharides that may be used in producing an aliphatic polyester resin include, but are not limited to, cellulose, cellulose nitrate, cellulose acetate, methyl cellulose, cellulose acetate, methyl cellulose, ethyl cellulose, carboxylmethyl cellulose, nitrocellulose, regenerated cellulose, viscose rayon or cupra, hemicellulose, starch, amylopectin, dextrin, dextran, glycogen, pectin, chitin, chitosan, derivatives thereof, and mixtures thereof. In one embodiment, cellulose acetate or ethyl cellulose can be utilized as the polyfunctional polysaccharide.

Some other semicrystalline polylactic acid material containing resin compositions may include an aliphatic polyester resin having a lactic acid component. Examples of a lactic acid component may include a homopolymer of lactic acid, a copolymer of different lactic acids (e.g., a copolymer of L-lactic acid and D-lactic acid, or a copolymer of a homopolymer of lactic acid and a lactate copolymer), or a copolymer of lactic acid and an aliphatic hydroxycarboxylic acid other than lactic acid.

The molecular weight of the semicrystalline polylactic acid material can be chosen to provide desired physical properties for the microporous articles that are formed using the semicrystalline polylactic acid materials. Therefore, the molecular weight of the semicrystalline polylactic acid material can vary as long as the microporous article such as, for example, a film, sheet, or web formed from a composition having two continuous phases has satisfactory physical properties. Generally, as the molecular weight of the semicrystalline polylactic acid material is decreased, the strength of the microporous article formed from a melt blended composition is reduced and the decomposition rate (e.g., biodegradability) is increased. As the molecular weight of the semicrystalline polylactic acid material is increased, the processability of the melt blended composition decreases, and forming (e.g., shaping) a microporous article from the composition of the melt blended composition may become more difficult.

A microporous article having sufficient elongation properties generally contains crystalline or semicrystalline polylactic acid material having a weight average molecular weight that is at least 3,000 g/mole (grams/mole), at least 10,000 g/mole, at least 50,000 g/mole, or at least 90,000 g/mole. The weight average molecular weight of semicrystalline polylactic acid material may be up to 5,000,000 g/mole, up to 2,500,000 g/mole, up to 1,000,000 g/mole, or up to 250,000 g/mole. In some embodiments, the weight average molecular weight of the semicrystalline polylactic acid material can be in a range of 3,000 g/mole to 5,000,000 g/mole, 10,000 g/mole to 2,500,000 g/mole, 50,000 g/mole to 1,000,000 g/mole, or 90,000 g/mole to 250,000 g/mole.

The amount of crystalline or semicrystalline polylactic acid material included in the initial composition is selected to provide a microporous article having desired properties as described above. The crystalline or semicrystalline polylactic acid material may have a concentration of at least 40 weight percent, at least 45 weight percent, at least 50 weight percent, or at least 55 weight percent based on the total weight of the initial composition. The concentration of the semicrystalline polylactic acid material may be up to 80 weight percent, up to 70 weight percent, up to 65 weight percent, or up to 60 weight percent based on the total weight of the initial composition. In some embodiments, the crystalline or semicrystalline polylactic acid concentration may be in a range of 40 to 80 weight percent, 45 to 70 weight percent, 50 to 65 weight percent, or 55 to 65 weight percent based on the total weight of the initial composition.

The initial composition as described earlier also comprises a diluent. The diluent may be referred to as a solvent or a compounding additive in a melt blended composition. The diluent can be a solid or a liquid at room temperature.

Examples of diluents suitable for mixing and heating with a semicrystalline polymeric material include nonpolymeric aliphatic ester diluents. Nonpolymeric aliphatic ester diluents can be mixed with a nucleating agent and a semicrystalline polymeric material, such as a semicrystalline polylactic acid material, to form a single liquid phase at or above the melting temperature of the semicrystalline polylactic acid material. The nonpolymeric aliphatic ester diluent is present in the melt blended composition in a sufficient quantity to phase separate from the semicrystalline polylactic acid material in the melt blended composition as the melt blended composition cools forming a composition having two continuous phases. The nonpolymeric aliphatic ester diluent phase can separate on cooling at or below the crystallization temperature of the semicrystalline polylactic acid material of the melt blended composition. The nonpolymeric aliphatic ester diluent may be washed or removed from the composition.

The nonpolymeric aliphatic ester diluent may have a boiling point at atmospheric pressure that is at least as high as the melting temperature of the semicrystalline polylactic acid material. Some nonpolymeric aliphatic ester diluents having lower boiling points may be used, however, if the nonpolymeric aliphatic ester diluent undergoes less than a 10 percent weight loss at the melting temperature of the semicrystalline polylactic acid material. Lower boiling point nonpolymeric aliphatic ester diluents may be used under super-atmospheric pressure to elevate the boiling point to a temperature at least as high as the melting temperature of the semicrystalline polylactic acid material.

Some examples of nonpolymeric aliphatic ester diluents include glycerol esters of Formula I.

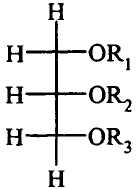

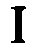

R₁ in Formula I can be an acyl functional group having 6 to 24 carbon atoms. An acyl functional group can be defined as R(C=O)-, and R is usually an alkyl group having one or more carbon atoms. R₂ in Formula I can be hydrogen, or an acyl functional group having 2 to 6 carbon atoms. R₃ in Formula I can be hydrogen, or an acyl functional group having 2 to 6 carbon atoms. In one embodiment, the nonpolymeric aliphatic ester diluent of Formula I is glycerin diacetomonocaprylate (i.e., each R₁ is an acyl having 8 carbon atoms, and R₂ and R₃ are each an acyl having 2 carbon atoms). In another embodiment, the nonpolymeric aliphatic ester diluent of Formula I is glycerin diacetomonocaprate (i.e., each R₁ is an acyl having 10 carbon atoms, and R₂ and R₃ are each an acyl having 2 carbon atoms). In a further embodiment, the nonpolymeric aliphatic ester diluent of Formula I is glycerin diacetomonooleate (i.e., each R₁ is an acyl having 18 carbon atoms, and R₂ and R₃ are each an acyl having 2 carbon atoms).

Other examples of nonpolymeric aliphatic ester diluents include alkylene fatty acid esters of Formula II.

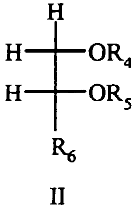

R₄ in Formula II can be an acyl functional group having 6 to 24 carbon atoms. R₅ in Formula II can be hydrogen, or an acyl functional group having 2 to 6 carbon atoms. R₆ in Formula II can be an alkyl functional group having 1 to 4 carbon atoms. In one embodiment, the nonpolymeric aliphatic ester diluent of Formula II is propylene glycol monocaprylate (i.e., each R₄ is an acyl having 8 carbon atoms, each R₅ is hydrogen, and each R₆ is an alkyl having 2 carbon atoms).

Still other examples of nonpolymeric aliphatic ester diluents include citrate esters of Formula III.

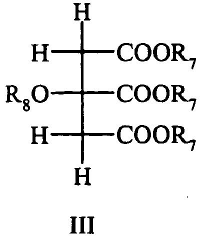

R₇ in Formula III independently can be hydrogen, or an alkyl functional group having 1 to 8 carbon atoms. R₈ in Formula III can an acyl functional group having 2 to 5 carbon atoms. In one embodiment, the nonpolymeric aliphatic ester diluent of Formula III is tributylacetyl citrate (i.e., each R₇ is an alkyl having 4 carbon atoms, and each R₈ is an acyl having 2 carbon atoms).

The nonpolymeric aliphatic ester diluent used in preparing the initial composition may have a concentration that is at least 20 weight percent, at least 25 weight percent, or at least 30 weight percent based on the total weight of the initial composition. The concentration of the nonpolymeric aliphatic ester diluent may be up to 60 weight percent, up to 55 weight percent, or up to 50 weight percent based on the total weight of the initial composition. In some embodiments, the nonpolymeric aliphatic ester diluent concentration may be in a range of 20 to 60 weight percent, 25 to 55 weight percent, or 30 to 50 weight percent based on the total weight of the initial composition. Mixtures or blends of the nonpolymeric aliphatic ester diluents of Formulas I - III may be used with a semicrystalline polylactic acid material and a nucleating agent for preparing an initial composition.

Nucleating agents are materials added to the initial composition that function as a foreign body when the melt blended composition is cooled. When a semicrystalline polymeric material is heated above its melting temperature, and then cooled below its crystallization temperature, the loosely coiled polymer chains orient themselves about the foreign body into regions of a three dimensional crystal pattern mixed with the amorphous polymer. The nucleating agent functions as an initiating site for polymeric material crystals in the melt blended composition. A greater number of small polymeric material crystals are formed when a nucleating agent is present.

The nucleating agent induces crystallization of the molten polymeric material from a single liquid state in the melt blended composition, and enhances the initiation of polymeric material crystallization sites to speed up crystallization of the polymeric material during cooling. The nucleating agent may be a solid, a semi-solid gel, or a discrete liquid droplet at the crystallization temperature of the polymeric material.

A nucleating agent is combined with a crystalline or semicrystalline polylactic acid material, and a nonpolymeric aliphatic ester diluent for preparing an initial composition. The initial composition is heated and mixed to at least the melting temperature of the crystalline or semicrystalline polylactic acid material to form a melt blended composition; the nucleating agent can be uniformly dispersed or dissolved in a single liquid phase of the melt blended composition. The single liquid phase comprising the polylactic acid material and the nonpolymeric aliphatic ester diluent can exist above the melting temperature of the crystalline or semicrystalline polylactic acid material. As the melt blended composition is cooled, the nucleating agent may initiate crystallization of the polylactic acid material to form a crystalline or semicrystalline polylactic acid matrix. The crystalline or semicrystalline polylactic acid matrix forms a first phase, and the nonpolymeric aliphatic ester diluent forms a second phase that is not miscible with the first phase. The first phase and the second phase form a composition.

Polylactic acid material crystallizes slowly in a melt blended composition, and the crystallization may be accelerated by the presence of a nucleating agent. The size of the resulting polylactic acid material domains may be influenced by the nucleating agent, by the concentrations of the semicrystalline polylactic acid material and the nonpolymeric aliphatic ester diluent in the melt blended composition, and by the processing conditions used for forming the composition. A polylactic acid material having smaller domains may result in the formation of more domains having an increase in the number of fibrils per unit volume. A network of interconnected micropores can be formed by stretching the co-composition in at least one direction. Stretching of the microporous article (e.g., a film) formed using a nucleating agent may result in the length of the fibrils being increased in comparison to a microporous article formed without a nucleating agent.

Some examples of nucleating agents include inorganic particles, organic compounds, organic acid salts, and imides. Examples of inorganic nucleating agents include, but not limited to, include, talc, clay, mica, calcium silicate, calcium titanate, and boron nitride. Examples of organic compounds include, but not limited to, propylene carbonate, a stereocomplex of poly L- and D-lactic acid, isotactic polypropylene, and low molecular weight poly(butylene terephthalate). Some examples of organic metallic compounds (e.g., pigments) include, but not limited to, copper phthalocyanine, chromophthal blue A3R pigment, and γ-quinacridone. Some examples of organic acid salts include, but not limited to, zinc phenylphosphonate, sodium salt of saccharin, sodium salt of bicyclo[2.2.1]heptane-2,3-dicarboxylic acid, and sodium benzoate. In some embodiments, the nucleating agent comprises copper phthalocyanine or zinc phenylphosphonate. In other embodiments, the nucleating agent comprises isotactic polypropylene, a mixture of isotactic polypropylene and zinc phenylphosphonate, a mixture of isotactic polypropylene and copper phthalocyanine, or combinations thereof.

The amount of a nucleating agent present in a melt blended composition may influence the rate of crystallization and the number of polylactic acid material domains formed. Smaller amounts of nucleating agent are generally needed for developing composition having two continuous phases capable of forming interconnected micropores. The concentration of the nucleating agent may be at least 0.01 weight percent, at least 0.1 weight percent, at least 0.5 weight percent, or at least 2 weight percent based on the total weight of the initial composition. The concentration of the nucleating agent may be up to 10 weight percent, up to 9 weight percent, up to 8 weight percent, or up to 7 weight percent based on the total weight of the initial composition. In some initial compositions, the concentration of the nucleating agent may be in a range of 0.01 to 10 weight percent, 0.1 to 9 weight percent, 0.5 to 8 weight percent, or 2 to 7 weight percent base on the total weight of the initial composition.

In some embodiments, an antimicrobial component can be added to the initial composition for forming antimicrobial microporous articles.

The antimicrobial component content in the initial composition is typically equal to or less than 5 wt. % of the initial composition. In some initial compositions, the antimicrobial component is less than 4.5 wt. %, less than 4.0 wt. %, less than 3.0 wt. %, or less than 2.0 wt. % of the initial composition.

The antimicrobial component may include one or more fatty acid esters of a polyhydric alcohol, fatty ethers of a polyhydric alcohol, or alkoxylated derivatives thereof (of either or both of the ester and/or ether), or combinations thereof. More specifically, the antimicrobial component is selected from the group consisting of a (C₇-C₁₂)saturated fatty acid ester of a polyhydric alcohol (preferably, a (C₈-C₁₂)saturated fatty acid ester of a polyhydric alcohol), an (C₈-C₂₂) unsaturated fatty acid ester of a polyhydric alcohol (preferably, an (C₁₂-C₂₂) unsaturated fatty acid ester of a polyhydric alcohol), a (C₇-C₁₂) saturated fatty ether of a polyhydric alcohol (preferably, a (C₈-C₁₂) saturated fatty ether of a polyhydric alcohol), an (C₈-C₂₂) unsaturated fatty ether of a polyhydric alcohol (preferably, an (C₁₂-C₂₂) unsaturated fatty ether of a polyhydric alcohol), an alkoxylated derivative thereof, and combinations thereof. Preferably, the esters and ethers are monoesters and monoethers, unless they are esters and ethers of sucrose in which case they can be monoesters, diesters, monoethers, or diethers. Various combinations of monoesters, diesters, monoethers, and diethers can be used in the initial composition of the present application. In some embodiments, esters and ethers having mono-, or di-, or tri- derivatives, as described above, may comprise antimicrobial activity, but should be avoided to the extent that they render the article or composition insufficiently antimicrobial.

Useful fatty acid esters of a polyhydric alcohol may have the formula:

(R¹-C(O)-O)ₙ-R²

wherein R¹ is the residue of a (C₇-C₁₂) saturated fatty acid (preferably, a (C₈-C₁₂) saturated fatty acid), or a (C₈-C₂₂) unsaturated (preferably, a C₁₂-C₂₂) unsaturated, including polyunsaturated) fatty acid, R² is the residue of a polyhydric alcohol (typically and preferably, glycerin, propylene glycol, and sucrose, although a wide variety of others can be used including pentaerythritol, sorbitol, mannitol, xylitol, etc.), and n = 1 or 2. The R² group includes at least one free hydroxyl group (preferably, residues of glycerin, propylene glycol, or sucrose). Preferred fatty acid esters of polyhydric alcohols are esters derived from C₇, C₈, C₉, C₁₀, C₁₁, and C₁₂ saturated fatty acids. For embodiments in which the polyhydric alcohol is glycerin or propylene glycol, n = 1, although when it is sucrose, n = 1 or 2. In general, monoglycerides derived from C₁₀ to C₁₂ fatty acids are food grade materials and GRAS materials.

Fatty acid esters are particularly useful candidates for treating food, and surfaces exposed to food, to reduce the number of human pathogens and spoilage in food since many of the monoesters have been reported to be food grade, generally recognized as safe (GRAS) materials, and have been reported to be effective as food preservatives and topical pharmaceutical agents. For example, Kabara, J. of Food Protection, 44:633-647 (1981) and Kabara, J. of Food Safety, 4:13-25 (1982) report that LAURICIDIN (the glycerol monoester of lauric acid commonly referred to as monolaurin), a food grade phenolic and a chelating agent may be useful in designing food preservative systems. Lauroyl ethylarginate is also approved by the FDA for use in foods.

Fatty acid monoesters, such as glycerol monoesters of lauric, caprylic, capric, and heptanoic acid and/or propylene glycol monoesters of lauric, caprylic, capric and heptanoic acid, are active against Gram positive bacteria, fungi, yeasts and lipid coated viruses but alone are not generally active against Gram negative bacteria. When the fatty acid monoesters are combined with the enhancers described below in the initial composition, the antimicrobial microporous article is active against Gram negative bacteria.

Certain of the antimicrobial components (e.g. fatty acid monoesters) can plasticize the aliphatic polyester. Exemplary fatty acid monoesters include, but are not limited to, glycerol monoesters of lauric (monolaurin), caprylic (monocaprylin), and capric (monocaprin) acid, and propylene glycol monoesters of lauric, caprylic, and capric acid, as well as lauric, caprylic, and capric acid monoesters of sucrose. Other fatty acid monoesters include glycerin and propylene glycol monoesters of oleic (18:1), linoleic (18:2), linolenic (18:3), and arachonic (20:4) unsaturated (including polyunsaturated) fatty acids. As is generally known, 18:1, for example, means the compound has 18 carbon atoms and 1 carbon-carbon double bond. Preferred unsaturated chains have at least one unsaturated group in the cis isomer form. In certain preferred embodiments, the fatty acid monoesters that are suitable for use in the initial composition include known monoesters of lauric, caprylic, and capric acid, such as that known as GML or the trade designation LAURICIDIN (the glycerol monoester of lauric acid commonly referred to as monolaurin or glycerol monolaurate), glycerol monocaprate, glycerol monocaprylate, propylene glycol monolaurate, propylene glycol monocaprate, propylene glycol monocaprylate, and combinations thereof.

Exemplary fatty acid diesters of sucrose include, but are not limited to, lauric, caprylic, and capric diesters of sucrose as well as combinations thereof.

A fatty ether of a polyhydric alcohol is preferably of the formula:

(R³-O)ₙ-R⁴,

wherein R³ is a (C₇-C₁₂)saturated aliphatic group (preferably, a (C₈-C₁₂) saturated aliphatic group), or a (C₈-C₂₂) unsaturated (preferably, (C₁₂-C₂₂) unsaturated, including polyunsaturated) aliphatic group, R⁴ is the residue of a polyhydric alcohol. Preferred polyhydric alcohols include glycerin, sucrose, or propylene glycol. For glycerin and propylene glycol n = 1, and for sucrose n = 1 or 2. Preferred fatty ethers are monoethers of (C₇-C₁₂) alkyl groups (more preferably, (C₈-C₁₂) alkyl groups).

Exemplary fatty monoethers include, but are not limited to, laurylglyceryl ether, caprylglycerylether, caprylylglyceryl ether, laurylpropylene glycol ether, caprylpropyleneglycol ether, and caprylylpropyleneglycol ether. Other fatty monoethers include glycerin and propylene glycol monoethers of oleyl (18:1), linoleyl (18:2), linolenyl (18:3), and arachonyl (20:4) unsaturated and polyunsaturated fatty alcohols. In certain preferred embodiments, the fatty monoethers that are suitable for use in the initial composition include laurylglyceryl ether, caprylglycerylether, caprylyl glyceryl ether, laurylpropylene glycol ether, caprylpropyleneglycol ether, caprylylpropyleneglycol ether, and combinations thereof. Unsaturated chains preferably have at least one unsaturated bond in the cis isomer form.

The alkoxylated derivatives of the aforementioned fatty acid esters and fatty ethers (e.g., one which is ethoxylated and/or propoxylated on the remaining alcohol groups) also have antimicrobial activity as long as the total alkoxylate is kept relatively low. Preferred alkoxylation levels are disclosed in U.S. Patent 5,208,257. If the esters and ethers are ethoxylated, total moles of ethylene oxide are preferably less than 5, more preferably less than 2.

The fatty acid esters or fatty ethers of polyhydric alcohols can be alkoxylated, preferably ethoxylated and/or propoxylated, by conventional techniques. Alkoxylating compounds are preferably selected from the group consisting of ethylene oxide, propylene oxide, and mixtures thereof, and similar oxirane compounds.

The initial compositions typically include a total amount of fatty acid esters, fatty ethers, alkoxylated fatty acid esters, or alkoxylated fatty ethers equal to or no greater than 5 weight % based on the total weight of the initial composition. In some initial compositions, the total amount of fatty acid esters, fatty ethers, alkoxylated fatty acid esters, or alkoxylated fatty ethers is less than 4.5 wt. %, less than 4.0 wt. %, less than 3.0 wt. %, or less than 2.0 wt. % of the initial composition.

Compositions of the present disclosure that include one or more fatty acid monoesters, fatty monoethers, hydroxyl acid esters of alcohols or alkoxylated derivatives thereof can also include a small amount of a di- or tri-fatty acid ester (i.e., a fatty acid di- or tri-ester), a di- or tri-fatty ether (i.e., a fatty di- or tri-ether), or alkoxylated derivative thereof. Preferably, such components comprise no more than 10 wt. %, no more than 7 wt. %, no more than 6 wt. %, or no more than 5 wt. %, of the total weight of the antimicrobial component. Thus, the monoester purity of the fatty acid monoester, fatty monoethers, hydroxyl acid esters of alcohols or alkoxylated derivatives thereof should exceed 85 %, preferably 90 %, and more preferably 95 %. For example, for monoesters, monoethers, or alkoxylated derivatives of glycerin, preferably there is no more than 10 wt. %, no more than 7 wt. %, no more than 6 wt. %, or no more than 5 wt. % of a diester, diether, triester, triether, or alkoxylated derivatives thereof present, based on the total weight of the antimicrobial-(monoester or monoether) components present in the initial composition. Preferably, the triester or diester content is kept low to preserve the antimicrobial efficacy of the antimicrobial component.

An additional class of antimicrobial component is a fatty alcohol ester of a hydroxyl functional carboxylic acid preferably of the formula:

R⁵-O-(-C(O)-R⁶-O)ₙH,

wherein R⁵ is the residue of a (C₇-C₁₄)saturated alkyl alcohol (preferably, a (C₇-C₁₂) saturated alkyl alcohol, more preferably, a (C₈-C₁₂) saturated alkyl alcohol) or a (C₈-C₂₂) unsaturated alcohol (including polyunsaturated alcohol), R⁶ is the residue of a hydroxycarboxylic acid wherein the hydroxycarboxylic acid has the following formula:

R⁷(CR⁸OH)ₚ(CH₂)_{q}COOH,

wherein: R⁷ and R⁸ are each independently H or a (C₁-C₈) saturated straight, branched, or cyclic alkyl group, a (C₆-C₁₂) aryl group, or a (C₆-C₁₂) aralkyl or alkaryl group wherein the alkyl groups are saturated straight, branched, or cyclic, wherein R⁷ and R⁸ may be optionally substituted with one or more carboxylic acid groups; p = 1 or 2; and q = 0-3; and n = 1, 2, or 3. The R⁶ group may include one or more free hydroxyl groups but preferably is free of hydroxyl groups. Preferred fatty alcohol esters of hydroxycarboxylic acids are esters derived from branched or straight chain C₈, C₉, C₁₀, C₁₁, or C₁₂ alkyl alcohols. The hydroxyacids typically have one hydroxyl group and one carboxylic acid group.

In one aspect, the antimicrobial component includes a (C₇-C₁₄) saturated fatty alcohol monoester of a (C₂-C₈) hydroxycarboxylic acid (preferably, a (C₇-C₁₂) saturated fatty alcohol monoester of a (C₂-C₈) hydroxycarboxylic acid, more preferably a (C₈-C₁₂) saturated fatty alcohol monoester of a (C₂-C₈) hydroxycarboxylic acid), a (C₈-C₂₂) mono- or poly-unsaturated fatty alcohol monoester of a (C₂-C₈) hydroxycarboxylic acid, an alkoxylated derivative of either of the foregoing, or combinations thereof. The hydroxycarboxylic acid moiety can include aliphatic and/or aromatic groups. For example, fatty alcohol esters of salicylic acid are possible. As used herein, a "fatty alcohol" is an alkyl or alkylene monofunctional alcohol having an even or odd number of carbon atoms.

Exemplary fatty alcohol monoesters of hydroxycarboxylic acids include, but are not limited to, (C₆-C₁₂) fatty alcohol esters of lactic acid such as octyl lactate, 2-ethylhexyl lactate (Purasolv EHL from Purac, Lincolnshire IL, lauryl lactate (Chrystaphyl 98 from Chemic Laboratories, Canton MA), lauryl lactyl lactate, 2-ethylhexyl lactyl lactate; (C8-C12) fatty alcohol esters of glycolic acid, lactic acid, 3-hydroxybutanoic acid, mandelic acid, gluconic acid, tartaric acid, and salicylic acid.

The alkoxylated derivatives of the fatty alcohol esters of hydroxy functional carboxylic acids (e.g., one which is ethoxylated and/or propoxylated on the remaining alcohol groups) also have antimicrobial activity as long as the total alkoxylate is kept relatively low. The preferred alkoxylation level is less than 5 moles, and more preferably less than 2 moles, per mole of hydroxycarboxylic acid.

The above antimicrobial components comprising an ester linkage are hydrolytically sensitive, and may be degraded by exposure to water, particularly at extreme pH (less than 4 or more than 10) or by certain bacteria that can enzymatically hydrolyze the ester to the corresponding acid and alcohol, which may be desirable in certain applications. For example, a microporous article may be made to degrade rapidly by incorporating an antimicrobial component comprising at least one ester group. If extended persistence of the microporous article is desired, an antimicrobial component, free of hydrolytically sensitive groups, may be used. For example, the fatty monoethers are not hydrolytically sensitive under ordinary processing conditions, and are resistant to microbial attack.

Another class of antimicrobial components includes cationic amine antimicrobial compounds, which include antimicrobial protonated tertiary amines and small molecule quaternary ammonium compounds. Exemplary small molecule quaternary ammonium compounds include benzalkonium chloride and alkyl substituted derivatives thereof, di-long chain alkyl (C₈-C₁₈) quaternary ammonium compounds, cetylpyridinium halides and their derivatives, benzethonium chloride and its alkyl substituted derivatives, octenidine and compatible combinations thereof.

Cationic antiseptics and disinfectants useful as the antimicrobial component include small molecule quaternary ammonium compounds, typically comprising one or more quaternary ammonium group having attached thereto at least one C₆ - C₁₈ linear or branched alkyl or aralkyl chain. Suitable compounds include those disclosed in Lea & Febiger, Chapter 13 in Block, S., Disinfection, Sterilization and Preservation, 4th ed., 1991 and may have the formula:

R⁹R¹⁰NR¹¹R¹²⁺X⁻

in which R⁹ and R¹⁰ are C₁-C₁₈ linear or branched alkyl, alkaryl, or aralkyl chains that may be substituted by N, O or S, R¹¹ and R¹² are C₁-C₆ alkyl, phenyl, benzyl or C₈-C₁₂ alkaryl groups, or R¹¹ and R¹² may form a ring such as a pyridine ring with the N of the quaternary ammonium group, X is an anion, preferably halide such as Cl⁻ or Br⁻ but possibly methosulfate, ethosulfate, phosphate or similar anions. Compounds within this class are: monoalkyltrimethylammonium salts, monoalkyldimethyl-benzyl ammonium salts, dialkyldimethyl ammonium salts, benzethonium chloride, alkyl substituted benzethonium halides such as methylbenzethonium chloride and octenidine.

Examples of quaternary ammonium antimicrobial components are: benzalkonium halides having an alkyl chain length of C₈-C₁₈, preferably C₁₂-C₁₆, more preferably a mixture of chain lengths, e.g., benzalkonium chloride comprising 40% C₁₂ alkyl chains, 50% C₁₄ alkyl chains, and 10% C₁₆ chains (available as Barquat MB-50 from Lonza Group Ltd., Basel, Switzerland); benzalkonium halides substituted with alkyl groups on the phenyl ring (available as Barquat 4250); dimethyldialkylammonium halides having C₈-C₁₈ alkyl groups, or mixtures of such compounds (available as Bardac 2050, 205M and 2250 from Lonza); and cetylpyridinium halides such as cetylpyridinium chloride (Cepacol Chloride available as Cepacol Chloride from Merrell Labs); benzethonium halides and alkyl substituted benzethonium halides (available as Hyamine 1622 and Hyamine 10X from Rohm and Haas).

A useful class of cationic antimicrobial components is based on protonated tertiary amines. Preferred cationic antimicrobial protonated tertiary amines have at least one C₆-C₁₈ alkyl group. Within this class are biodegradable derivatives of amino acids, as described in PCT publications WO 01/94292, WO 03/013454 and WO 03/034842, and combinations of those with sodium sorbate, potassium sorbate or sorbic acid, see WO 02/087328. These cationic antimicrobial components can be degraded in the environment or on living tissue. WO 03/013454 teaches such antimicrobial components having the formula 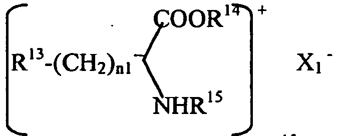 in which X may be Br⁻, Cl⁻ or HSO₄⁻, R¹⁵ may be a straight C₈-C₁₄ alkyl chain from an acid, e.g., saturated fatty hydroxy acid, R¹⁴ is a C₁-C₁₈ straight chain or branched alkyl or an aromatic moiety; and R¹³ may be -NH₃, 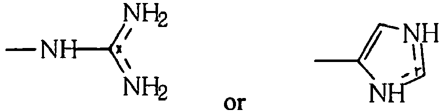 and n1 may be 0 - 4.

One useful member of this class of materials is lauroylethylarginate (the ethyl ester and lauric acid amide of the amino acid arginine (available as Mirenat N from A&B Ingredients, Fairfield, New Jersey)). Methods for producing these compositions are disclosed in WO 01/94292.

Cationic antimicrobial components are typically added to the initial compositions at a concentration equal to or no greater than 5 weight % based on the total weight of the initial composition. In some initial compositions, the total amount of cationic antimicrobial components is less than 4.5 wt. %, less than 4.0 wt. %, less than 3.0 wt. %, or less than 2.0 wt. % of the initial composition. Lower levels may be possible when used in combination with certain enhancers such as sorbic acid and/or its salts.

The antimicrobial components of this disclosure may be used alone or in combination in order to effectively kill microorganisms. Combinations of antimicrobial components that result in unstable compositions, or that are incompatible with each other should be avoided. For example, quaternary ammonium compounds may be incompatible with alkyl carboxylic acids or surfactants containing a sulfate moiety and/or sulfonic acid, and certain salts may cause precipitation of quaternary ammonium compounds.

In certain embodiments, antimicrobial components are non-ionic and have a hydrophile/lipophile balance (HLB) of at most 6.2, at most 5.8, or at most 5.5. Other preferred ranges for HLB are at least 3, least 3.2, or at least 3.4. The HLB may be determined from using the functional group contribution calculation shown in Surfactant Systems, Attwood, Chapman and Hall, London, 1983.

Certain antimicrobial components are uncharged and have an alkyl or alkenyl hydrocarbon chain containing at least 7 carbon atoms. For melt processing, preferred antimicrobial components have low volatility and do not decompose under process conditions. The preferred antimicrobial components contain less than 2 wt. % water, and more preferably less than 0.10 wt. % (determined by Karl Fischer analysis). Moisture content is kept low in order to prevent hydrolysis of the semicrystalline polylactic acid material and to give clarity to extruded film. The moisture level should be similarly controlled for solvent cast films that are dried at elevated temperatures, e.g. greater than 50°C- 60°C.

As used herein, the term "antiseptic" refers to a substance that inhibits growth and reproduction of disease-causing microorganisms, especially those substances that may contact mammalian tissue such as skin, wounds, mucosal tissue and the like. In most cases, "antiseptic" is synonymous with antimicrobial when used to control mammalian pathogens. Antiseptics and antimicrobial components described herein may be used alone, in combination, or with other antimicrobial components. Additional antimicrobial components for use with those already described include peroxides, C₆-C₁₄ alkyl carboxylic acids and alkyl ester carboxylic acids, antimicrobial natural oils, polymeric biguanides (such as polyhexamethylene biguanide) and bisbiguanides (such as chlorhexidine and its salts including chlorhexidine gluconate) and compatible combinations thereof as mentioned in U.S. Patent Publication 20060051384. Other compatible antiseptics that may be used in combination with the initial compositions on surfaces are iodine, iodophors, antimicrobial metals and metal salts such as silver salts and silver oxide, copper and zinc salts. In addition, certain antibiotics may be blended into the initial compositions or coated on the surface of articles comprising them and include Neosporin, polymyxin, bacitracin, mupirocin, rifampin, minocycline, tetracycline, beta lactam antibiotics such as penicillin, methicillin and amoxicillin, fluoroquinolones, clindamycin, cephalosporins, macrolides, and aminoglycosides.

The initial compositions described herein may further include an enhancer (preferably a synergist) to enhance the antimicrobial activity especially against Gram negative bacteria, e.g., *E*. *coli* and *Pseudomonas sp.* The chosen enhancer preferably affects the cell envelope of the bacteria. While not bound by theory, it is presently believed that the enhancer functions by allowing the antimicrobial component to more easily enter the cell cytoplasm and/or by facilitating disruption of the cell envelope. The enhancer component may include an alpha-hydroxy acid, a beta-hydroxy acid, other carboxylic acids, a (C₂-C₆) saturated or unsaturated alkyl carboxylic acid, a (C₆-C₁₆) aryl carboxylic acid, a (C₆-C₁₆) aralkyl carboxylic acid, a (C₆-C₁₂) alkaryl carboxylic acid, a phenolic compound (such as certain antioxidants and parabens), a (C₅-C₁₀) monohydroxy alcohol, a chelating agent, a glycol ether (i.e., ether glycol), or oligomers that degrade to release one of the above enhancers. Examples of such oligomers are oligomers of glycolic acid, lactic acid or both having at least 6 repeat units. Various combinations of enhancers can be used if desired.

The alpha-hydroxy acid, beta-hydroxy acid, and other carboxylic acid enhancers are preferably present in their protonated, free acid form. It is not necessary for all of the acidic enhancers to be present in the free acid form; however, the preferred concentrations listed below refer to the amount present in the free acid form. Additional, non-alpha hydroxy acid, betahydroxy acid or other carboxylic acid enhancers may be added in order to acidify the formulation or buffer it at a pH to maintain antimicrobial activity. Preferably, acids are used having a pKa greater than about 2.5, preferably greater than about 3, and most preferably greater than about 3.5 in order to avoid hydrolyzing the aliphatic polyester component. Furthermore, chelator enhancers that include carboxylic acid groups are preferably present with at least one, and more preferably at least two, carboxylic acid groups in their free acid form. The concentrations given below assume this to be the case. The enhancers in the protonated acid form are believed to not only increase the antimicrobial efficacy, but to improve compatibility when incorporated into the aliphatic polyester component.

One or more enhancers may be used in the compositions of the present invention at a suitable level to produce the desired result. Enhancers are typically present in a total amount equal to or less than 5 wt. % based on the total weight of the initial composition. In some initial compositions, the total amount of enhancers is less than 4.5 wt. %, less than 4.0 wt. %, less than 3.0 wt. %, or less than 2.0 wt. % of the initial composition. Such concentrations typically apply to alpha-hydroxy acids, beta-hydroxy acids, other carboxylic acids, chelating agents, phenolics, ether glycols, and (C₅-C₁₀) monohydroxy alcohols.

The ratio of the enhancer component relative to the total concentration of the antimicrobial component is preferably within a range of 10:1 to 1:300, and more preferably 5:1 and 1:10, on a weight basis.

The alpha-hydroxy acid is typically a compound of the formula:

R¹⁶(CR¹⁷OH)ₙ₂COOH

wherein: R¹⁶ and R¹⁷ are each independently H or a (C₁-C₈) alkyl group (straight, branched, or cyclic), a (C₆-C₁₂) aryl, or a (C₆-C₁₂) aralkyl or alkaryl group (wherein the alkyl group is straight, branched, or cyclic), R¹⁶ and R¹⁷ may be optionally substituted with one or more carboxylic acid groups; and n2 = 1-3, preferably, n2 = 1-2.

Exemplary alpha-hydroxy acids include, but are not limited to, lactic acid, malic acid, citric acid, 2-hydroxybutanoic acid, 3-hydroxybutanoic acid, mandelic acid, gluconic acid, glycolic acid, tartaric acid, alpha-hydroxyethanoic acid, ascorbic acid, alpha-hydroxyoctanoic acid, and hydroxycaprylic acid, as well as derivatives thereof (e.g., compounds substituted with hydroxyls, phenyl groups, hydroxyphenyl groups, alkyl groups, halogens, as well as combinations thereof). Preferred alpha-hydroxy acids can include lactic acid, malic acid, and mandelic acid. These acids may be in D, L, or DL form and may be present as free acid, lactone, or partial salts thereof. All such forms are encompassed by the term "acid." Preferably, the acids are present in the free acid form. In certain preferred embodiments, the alpha-hydroxy acids useful in the initial compositions of the present invention are selected from the group consisting of lactic acid, mandelic acid, malic acid, and mixtures thereof. Other suitable alpha-hydroxy acids are described in U.S. Patent No. 5,665,776 (Yu).

One or more alpha-hydroxy acids may be incorporated in the inventive compositions, and/or applied to the surfaces of articles comprising the inventive composition, in an amount to produce the desired result. One or more alpha-hydroxy acids are typically present in a total amount equal to or less than 5 wt. % based on the total weight of the initial composition. In some initial compositions, they may be present in an amount less than 4.5 wt. %, less than 4.0 wt. %, less than 3.0 wt. %, or less than 2.0 wt. % of the initial composition.

The weight ratio of alpha-hydroxy acid enhancer to total antimicrobial component is at most 50:1, at most 30:1, at most 20:1, at most 10:1, at most 5:1 or at most 1:1. The ratio of alpha-hydroxy acid enhancer to total antimicrobial component may be at least 1:120, at least 1:80, or at least 1:60. Preferably the ratio of alpha-hydroxy acid enhancer to total antimicrobial component is within a range of 1:60 to 2:1.

A beta-hydroxy acid enhancer is typically a compound represented by the formula:

R¹⁸(CR¹⁹OH)ₙ₃(CHR²⁰)ₘCOOH

or 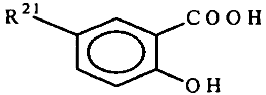 wherein: R¹⁸, R¹⁹, and R²⁰ are each independently H or a (C₁-C₈)alkyl group (saturated straight, branched, or cyclic group), (C₆-C₁₂) aryl, or (C₆-C₁₂) aralkyl or alkaryl group (wherein the alkyl group is straight, branched, or cyclic), R¹⁸ and R¹⁹ may be optionally substituted with one or more carboxylic acid groups; m = 0 or 1; n3 = 1-3 (preferably, n3 = 1-2); and R²¹ is H, (C₁-C₄) alkyl or a halogen.

Exemplary beta-hydroxy acids include, but are not limited to, salicylic acid, beta-hydroxybutanoic acid, tropic acid, and trethocanic acid. In certain preferred embodiments, the beta-hydroxy acids useful in the compositions of the present invention are selected from the group consisting of salicylic acid, beta-hydroxybutanoic acid, and mixtures thereof. Other suitable beta-hydroxy acids are described in U.S. Pat. No. 5,665,776.

One or more beta-hydroxy acids may be used in the compositions of the present invention at a suitable level to produce the desired result. They may be present in a total amount equal to or less than 5 wt. % based on the total weight of the initial composition. They may also be present in a total amount less than 4.5 wt. %, less than 4.0 wt. %, less than 3.0 wt. %, or less than 2.0 wt. % of the initial composition. Higher concentrations may become irritating to tissue. Alternatively, beta-hydroxy acids may be applied to the surface of articles comprising the inventive composition. When present on the surface, the levels may be 0.05 wt. %, preferably 0.1 wt. %, more preferably 0.25 wt. %, and most preferably 0.5 wt. % of the article.

The weight ratio of beta-hydroxy acid enhancer to total antimicrobial component is preferably at most 50:1, at most 30:1, at most 20:1, at most 10:1, at most 5:1, or at most 1:1. The ratio of beta-hydroxy acid enhancer to total antimicrobial component is preferably at least 1:120, at least 1:80, or at least 1:60. Preferably the ratio of beta-hydroxy acid enhancer to total antimicrobial component is within a range of 1:60 to 2:1, more preferably 1:15 to 1:1.

In systems with low concentrations of water, or that are essentially free of water, transesterification may be the principle route of loss of the fatty acid monoester and alkoxylated derivatives of these active ingredients and loss of carboxylic acid containing enhancers may occur due to esterification. Thus, certain alpha-hydroxy acids (AHA) and beta-hydroxy acids (BHA) are particularly preferred since these are believed to be less likely to transesterify the ester antimicrobial or other ester by reaction of the hydroxyl group of the AHA or BHA. For example, salicylic acid may be particularly preferred in certain formulations since the phenolic hydroxyl group is a much more acidic alcohol and thus much less likely to react. Other particularly preferred compounds in anhydrous or low-water content formulations include lactic, mandelic, malic, citric, tartaric, and glycolic acid. Benzoic acid and substituted benzoic acids that do not include a hydroxyl group, while not hydroxyl acids, are also preferred due to a reduced tendency to form ester groups. This applies to both melt and solvent cast processable systems or compositions.

Carboxylic acids other than alpha- and beta-carboxylic acids are suitable enhancers. They include alkyl, aryl, aralkyl, or alkaryl carboxylic acids typically having equal to or less than 12 carbon atoms. A preferred class of these can be represented by the following formula:

R²²(CR²³₂)ₙ₄COOH

wherein: R²² and R²³ are each independently H or a (C₁-C₄) alkyl group (which can be a straight, branched, or cyclic group), a (C₆-C₁₂) aryl group, a (C₆-C₁₂) group containing both aryl groups and alkyl groups (which can be a straight, branched, or cyclic group), R²² and R²³ may be optionally substituted with one or more carboxylic acid groups; and n4 = 0-3, preferably, n4 = 0-2. The carboxylic acid may be a (C₂-C₆) alkyl carboxylic acid, a (C₆-C₁₆) aralkyl carboxylic acid, or a (C₆-C₁₆) alkaryl carboxylic acid. Exemplary acids include, but are not limited to acetic acid, propionic acid, sorbic acid, benzoic acid, benzylic acid, and nonylbenzoic acid.

One or more such carboxylic acids may be used in the initial compositions of the present invention in amounts sufficient to produce the desired result. In certain embodiments, they are present in a total amount no greater than 5 wt. %, and preferably no greater than 3 wt. %, based on the total weight of the initial composition.

Alternatively, carboxylic acid enhancers may be present on the surface of a microporous article (e.g., antimicrobial) made from the initial composition. When present on the surface, the amounts used may be 0.05 wt. %, preferably 0.1 wt. %, more preferably 0.25 wt. %, and most preferably 0.5 wt. % of the article.

The weight ratio of the total concentration of carboxylic acids (other than alpha- or beta-hydroxy acids) to the total concentration of the antimicrobial component is preferably within a range of 10:1 to 1:100, and preferably 2:1 to 1:10.

In some embodiments, a chelating agent (i.e., chelator) as an enhancer, can be added to the initial composition. The chelating agent is typically an organic compound capable of multiple coordination sites with a metal ion in solution. Typically these chelating agents are polyanionic compounds and coordinate best with polyvalent metal ions. Exemplary chelating agents include, but are not limited to, ethylene diamine tetraacetic acid (EDTA) and salts thereof (e.g., EDTA(Na)₂, EDTA(Na)₄, EDTA(Ca), EDTA(K)₂), sodium acid pyrophosphate, acidic sodium hexametaphosphate, adipic acid, succinic acid, polyphosphoric acid, sodium acid pyrophosphate, sodium hexametaphosphate, acidified sodium hexametaphosphate, nitrilotris(methylenephosphonic acid), diethylenetriaminepentaacetic acid, 1-hydroxyethylene, 1,1-diphosphonic acid, and diethylenetriaminepenta-(methylenephosphonic acid). Certain carboxylic acids, particularly the alpha-hydroxy acids and beta-hydroxy acids, can also function as chelators, e.g., malic acid and tartaric acid.

Also, included as chelators are compounds highly specific for binding ferrous and/or ferric ion such as siderophores, and iron binding proteins. Iron binding proteins, include, for example, lactoferrin, and transferrin. Siderophores include, for example, enterochlin, enterobactin, vibriobactin, anguibactin, pyochelin, pyoverdin, and aerobactin.

In certain embodiments, the chelating agents useful in the initial compositions of the present disclosure include those selected from the group consisting of ethylenediaminetetraacetic acid and salts thereof, succinic acid, and mixtures thereof. Preferably, either the free acid or the mono- or di-salt form of EDTA is used.

One or more chelating agents may be used in the initial compositions of the present disclosure at a suitable level to produce the desired result. They may be used in amounts similar to the carboxylic acids described above.

The ratio of the total concentration of chelating agents (other than alpha- or beta-hydroxy acids) to the total concentration of the antimicrobial component is preferably within a range of 10:1 to 1:100, and more preferably 1:1 to 1:10, on a weight basis.

A phenolic compound enhancer is typically a compound having the following general structure: 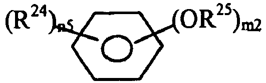 wherein: m2 is 0 to 3 (especially 1 to 3), n5 is 1 to 3 (especially 1 to 2), each R²⁴ independently is alkyl or alkenyl of up to 12 carbon atoms (especially up to 8 carbon atoms) optionally substituted with O in or on the chain (e.g., as a carbonyl group) or OH on the chain, and each R²⁵ independently is H or alkyl or alkenyl of up to 8 carbon atoms (especially up to 6 carbon atoms) optionally substituted with O in or on the chain (e.g., as a carbonyl group) or OH on the chain, but if R²⁵ is H, n5 preferably is 1 or 2.

Examples of phenolic enhancers include, but are not limited to, butylated hydroxy anisole, e.g., 3(2)-tert-butyl-4-methoxyphenol (BHA), 2,6-di-tert-butyl-4-methylphenol (BHT), 3,5-di-tert-butyl-4-hydroxybenzylphenol, 2,6-di-tert-4-hexylphenol, 2,6-di-tert-4-octylphenol, 2,6-di-tert-4-decylphenol, 2,6-di-tert-butyl-4-ethylphenol, 2,6-di-tert-4-butylphenol, 2,5-di-tert-butylphenol, 3,5-di-tert-butylphenol, 4,6-di-tert-butyl-resorcinol, methyl paraben (4-hydroxybenzoic acid methyl ester), ethyl paraben, propyl paraben, butyl paraben, 2-phenoxyethanol, as well as combinations thereof. One group of the phenolic compounds is the phenol species having the general structure shown above where R²⁵ is H and where R²⁴ is alkyl or alkenyl of up to 8 carbon atoms, and n4 is 0, 1, 2, or 3, especially where at least one R²⁴ is butyl and particularly tert-butyl, and especially the non-toxic members thereof being preferred. Some of the phenolic enhancers are synergists, such as BHA, BHT, methyl paraben, ethyl paraben, propyl paraben, and butyl paraben as well as combinations of these.

One or more phenolic compounds may be used in the initial compositions of the present disclosure at a suitable level to produce the desired result. The concentrations of the phenolic compounds may vary widely, but typically greater than 0.5 wt. %, based on the total weight of the composition, can be effective when the above-described esters are present within the above-noted ranges. In some embodiments, they are present in a total amount of at least 0.75 wt-%, or at least 1.0 wt. %, based on the total weight of the initial composition. In other embodiments, they are present in a total amount equal to or no greater than 5 wt. %, no greater than 4 wt. %, or no greater than 2 wt. %, based on the initial composition.

In commercially available PLA (poly(lactic acid)), antioxidant may be present, e.g., about 0.25 - 0.50 wt. %. When antioxidants are added to an aliphatic polyester, the antioxidants are believed to be uniformly mixed (and perhaps dissolved) within the material, with a minimal amount on the surface to enhance antimicrobial activity. Significantly lower concentrations of phenolics are typically employed for antioxidant use (e.g., not more than 0.1%) than are employed when using as an enhancer for the antimicrobial component (e.g., greater than 1%). The phenolic compounds may be present on the surface of the composition. When present on the surface, the levels may be at least 0.05 wt. %, preferably at least 0.1 wt. %, more preferably at least 0.25 wt. %, and most preferably at least 0.5 wt. % of the article to which they are applied.

The weight ratio of the total phenolic concentration to the total concentration of the antimicrobial component may be within a range of 1:1 to 1:100, or preferably within a range of 1:1 to 1:10, on a weight basis.

The above-noted concentrations of the phenolics are normally observed unless concentrated formulations for subsequent dilution are intended. The minimum concentration of the phenolics and the antimicrobial components to provide an antimicrobial effect will vary with the particular application.

An additional enhancer is a monohydroxy alcohol having 5-10 carbon atoms, including C₅-C₁₀ monohydroxy alcohols (e.g., octanol and decanol). In certain embodiments, alcohols useful in the compositions of the present invention are selected from the group n-pentanol, 2 pentanol, n-hexanol, 2 methylpentyl alcohol, n-octanol, 2-ethylhexyl alcohol, decanol, and mixtures thereof.

C₅-C₁₀ alcohols may be present in a total amount equal to or no greater than about 5 weight % based on the total weight of the initial composition. C₅-C₁₀ alcohols may be applied to the surface of articles comprising the composition of polymer and antimicrobial component. When present on the surface, amounts may be at least 0.05 wt. %, preferably at least 0.1 wt. %, more preferably at least 0.25 wt. %, and most preferably at least 0.5 wt. % of the article to which the composition is applied.

An additional enhancer is an ether glycol. Exemplary ether glycols include those of the formula:

R"'-O-(CH₂CHR""O)ₙ₆(CH₂CHR""O)H,

wherein R'" = H, a (C₁-C₈) alkyl, or a (C₆-C₁₂) aralkyl or alkaryl; and each R"" is independently = H, methyl, or ethyl; and n6 = 0-5, preferably 1-3. Examples include 2-phenoxyethanol, dipropylene glycol, triethylene glycol, the line of products available under the trade designation DOWANOL DB (di(ethylene glycol) butyl ether), DOWANOL DPM (di(propylene glycol)monomethyl ether), and DOWANOL TPnB (tri(propylene glycol) monobutyl ether), as well as many others available from Dow Chemical Company, Midland Michigan.

One or more ether glycols may be present in a total amount equal to or no greater than 5 weight percent based on the total initial composition. The ether glycols may be present on the surface of articles comprising the inventive composition. When present on the surface, the amounts may be at least 0.05 wt. %, preferably at least 0.1 wt. %, more preferably at least 0.25 wt. %, and most preferably at least 0.5 wt. % of the articles to which the glycols are applied as part of the inventive composition.

Oligomers that release an enhancer may be prepared by a number of methods. For example, oligomers may be prepared from alpha hydroxy acids, beta hydroxy acids, or mixtures thereof by standard esterification techniques. Typically, these oligomers have at least two hydroxy acid units, preferably at least 10 hydroxy acid units, and most preferably at least 50 hydroxy acid units. For example, a copolymer of lactic acid and glycolic acid may be prepared as shown in the Examples section.

Alternatively, oligomers of (C₂-C₆) dicarboxylic acids and diols may be prepared by standard esterification techniques. These oligomers preferably have at least 2 dicarboxylic acid units, preferably at least 10 dicarboxylic acid units, and most preferably at least 50 dicarboxylic acid units.

The enhancer releasing oligomeric polyesters used typically have a weight average molecular weight of less than 10,000 Daltons and preferably less than 8,000 Daltons.

These oligomeric polyesters may be hydrolyzed. Hydrolysis can be accelerated by an acidic or basic environment, for example at a pH less than 5 or greater than 8. The oligomers may be degraded enzymatically by enzymes present in the composition or in the environment in which it is used, for example from mammalian tissue or from microorganisms in the environment.

In some embodiments, initial compositions of the present disclosure can further include one or more surfactants to promote compatibility of the initial composition and to help wet the surface and/or to aid in contacting and killing microorganisms. As used herein the term "surfactant" means an amphiphile (a molecule possessing both polar and nonpolar regions which are covalently bound) capable of reducing the surface tension of water and/or the interfacial tension between water and an immiscible liquid. The term is meant to include soaps, detergents, emulsifiers, surface active agents, and the like. The surfactant can be cationic, anionic, nonionic, or amphoteric. In applications in which biodegradability is important, it may be desirable to incorporate biodegradable surfactants, which typically include ester and/or amide groups that may be hydrolytically or enzymatically cleaved. A variety of conventional surfactants may be used; however, certain ethoxylated surfactants can reduce or eliminate the antimicrobial efficacy of some of the antimicrobial lipid components.

The reason for this effect is not known and not all ethoxylated surfactants display this negative effect. For example, poloxamer (polyethylene oxide/polypropylene oxide) surfactants have been shown to be compatible with the antimicrobial lipid component, but ethoxylated sorbitan fatty acid esters such as those sold under the trade name TWEEN by ICI Americas, Inc. has not been compatible. It should be noted that these are broad generalizations and the activity could be formulation dependent. One skilled in the art can determine compatibility of a surfactant by making the formulation and testing for antimicrobial activity as described in the Examples herein. Combinations of various surfactants can be used.

Certain antimicrobial components are amphiphiles and may be surface active. For example, certain antimicrobial alkyl monoglycerides described herein are surface active. For certain embodiments of the invention, the antimicrobial lipid component is considered distinct from a surfactant component.

Surfactants that have an HLB (i.e., hydrophile to lipophile balance) of at least 4 or at least 8 are preferred. More preferred surfactants have an HLB of at least 12. Most preferred surfactants have an HLB of at least 15.

Examples of the various classes of surfactants are described below. In certain preferred embodiments, the surfactants useful in the initial compositions of the present disclosure are selected from the group consisting of sulfonates, sulfates, phosphonates, phosphates, poloxamers (polyethylene oxide/polypropylene oxide block copolymers), alkyl lactates, alkyl carboxylates, aralkyl carboxylates, alkylethoxylated carboxylates, aralkylethoxylated carboxylates, cationic surfactants, and mixtures thereof. In certain more preferred embodiments, the surfactants useful in the initial compositions of the present disclosure are selected from the group consisting of sulfonates, sulfates, phosphates, and mixtures thereof. In one aspect, the surfactant is selected from (C₈-C₂₂) alkyl sulfate salts (e.g., sodium salt), di(C₈-C₁₃ alkyl)sulfosuccinate salts, C₈-C₂₂ alkyl sarconsinate, and combinations thereof.

One or more surfactants may be used in and/or on the initial compositions of the present disclosure at a suitable level to produce the desired result. In some embodiments, when used in the initial composition, they are present in a total amount of at least 0.1 wt. %, at least 0.5 wt. %, or at least 1.0 wt. %, based on the total weight of the initial composition. In other embodiments, they are present in a total amount of no greater than 10 wt. %, no greater than 7.5 wt. %, no greater than 6 wt. %, or no greater than 4 wt. %, based on the total weight of the initial composition. The ratio of the total concentration of surfactant to the total concentration of the antimicrobial component may be within a range of 5:1 to 1:100, from 3:1 to 1:10, or from 2:1 to 1:3, on a weight basis. The surfactants may be present on the surface of an article comprising the initial composition. When present on the surface, amounts may be 0.05 wt. %, preferably 0.1 wt. %, more preferably 0.25 wt. %, and most preferably 0.5 wt. % of the microporous article to which the surfactant is applied.

Exemplary cationic surfactants include, but are not limited to, salts of optionally polyoxyalkylenated primary, secondary, or tertiary fatty amines; quaternary ammonium salts such as tetraalkylammonium, alkylamidoalkyltrialkylammonium, trialkylbenzylammonium, trialkylhydroxyalkylammonium, or alkylpyridinium halides (chlorides or bromides) as well as other anionic counterions, such as but not limited to, alkyl sulfates, such as but not limited to, methosulfate and ethosulfate; imidazoline derivatives; amine oxides of a cationic nature (e.g., at an acidic pH).

The cationic surfactants may be selected from the group consisting of tetralkyl ammonium, trialkylbenzylammonium, and alkylpyridinium halides as well as other anionic counterions, such as but not limited to, C₁-C₄ alkyl sulfates, such as but not limited to, methosulfate and ethosulfate, and mixtures thereof.

Amine oxide surfactants may be used including alkyl and alkylamidoalkyldialkylamine oxides of the following formula:

(R²⁶)₃-N→O,

wherein R²⁶ is a (C₁-C₃₀) alkyl group (preferably a (C₁-C₁₄) alkyl group) or a (C₆-C₁₈) aralklyl or alkaryl group, wherein any of these groups can be optionally substituted in or on the chain by N-, O-, or S-containing groups such as amide, ester, hydroxyl, and the like. Each R²⁶ may be the same or different provided at least one R²⁶ group includes at least eight carbons. Optionally, the R²⁶ groups can be joined to form a heterocyclic ring with the nitrogen to form surfactants such as amine oxides of alkyl morpholine, alkyl piperazine, and the like. In one such surfactant, two R²⁶ groups are methyl and one R²⁶ group is a (C₁₂-C₁₆)alkyl or alkylamidopropyl group. Examples of amine oxide surfactants include those commercially available under the trade designations AMMONYX LO, LMDO, and CO, which are lauryldimethylamine oxide, laurylamidopropyldimethylamine oxide, and cetyl amine oxide, all from Stepan Company (Northfield, Illinois).

Exemplary anionic surfactants include, but are not limited to, sarcosinates, glutamates, alkyl sulfates, sodium or potassium alkyleth sulfates, ammonium alkyleth sulfates, ammonium laureth-n-sulfates, laureth-n-sulfates, isethionates, glycerylether sulfonates, sulfosuccinates, alkylglyceryl ether sulfonates, alkyl phosphates, aralkyl phosphates, alkylphosphonates, and aralkylphosphonates. These anionic surfactants may have a metal or organic ammonium counterion. Certain useful anionic surfactants are selected from the group consisting of: sulfonates and sulfates such as alkyl sulfates, alkylether sulfates, alkyl sulfonates, alkylether sulfonates, alkylbenzene sulfonates, alkylbenzene ether sulfates, alkylsulfoacetates, secondary alkane sulfonates, secondary alkylsulfates, and the like. Many of these can be represented by the formulas:

R²⁶-(OCH₂CH₂)ₙ₇(OCH(CH₃)CH₂)ₚ₂-(Ph)ₐ-(OCH₂CH₂)ₘ₃-(O)_{b}-SO₃⁻M⁺

and

R²⁶-CH[SO₃-M⁺]-R²⁷

wherein: a and b = 0 or 1; n7, p2, and m3 = 0 - 100 (preferably 0 - 20); R²⁶ is defined as above provided at least one R²⁶ or R²⁷ is at least C8; R²⁷ is a (C₁-C₁₂)alkyl group (saturated straight, branched, or cyclic group) that may be optionally substituted by N, O, or S atoms or hydroxyl, carboxyl, amide, or amine groups; Ph = phenyl; and M is a cationic counterion such as H, Na, K, Li, ammonium, or a protonated tertiary amine such as triethanolamine or a quaternary ammonium group.

In the formula above, the ethylene oxide groups (i.e., the "n7" and "m3" groups) and propylene oxide groups (i.e., the "p2" groups) can occur in reverse order as well as in a random, sequential, or block arrangement. R²⁶ may be an alkylamide group such as R²⁸-C(O)N(CH₃)CH₂CH₂- as well as ester groups such as -OC(O)-CH₂- wherein R²⁸ is a (C₈-C₂₂)alkyl group (branched, straight, or cyclic group). Examples include, but are not limited to: alkyl ether sulfonates such as lauryl ether sulfates such as POLYSTEP B12 (n = 3-4, M = sodium) and B22 (n = 12, M = ammonium) available from Stepan Company, Northfield, IL and sodium methyl taurate (available under the trade designation NIKKOL CMT30 from Nikko Chemicals Co., Tokyo, Japan); secondary alkane sulfonates such as Hostapur SAS which is a Sodium (C14-C17)secondary alkane sulfonates (alpha-olefin sulfonates) available from Clariant Corp., Charlotte, NC; methyl-2-sulfoalkyl esters such as sodium methyl-2-sulfo(C₁₂-₁₆)ester and disodium 2-sulfo(C₁₂-C₁₆)fatty acid available from Stepan Company under the trade designation ALPHASTEP PC-48; alkylsulfoacetates and alkylsulfosuccinates available as sodium laurylsulfoacetate (under the trade designation LANTHANOL LAL) and disodiumlaurethsulfosuccinate (STEPANMILD SL3), both from Stepan Company; alkylsulfates such as ammoniumlauryl sulfate commercially available under the trade designation STEPANOL AM from Stepan Company; dialkylsulfosuccinates such as dioctylsodiumsulfosuccinate available as Aerosol OT from Cytec Industries.

Suitable anionic surfactants also include phosphates such as alkyl phosphates, alkylether phosphates, aralkylphosphates, and aralkylether phosphates. Many may be represented by the formula:

[R²⁶-(Ph)ₐ-O(CH₂CH₂O)ₙ₇(CH₂CH(CH₃)O)ₚ₂]_{q2}-P(O)[O⁻ M⁺]ᵣ,

wherein: Ph, R²⁶, a, n7, p2, and M are defined above; r is 0-2; and q2 = 1-3; with the proviso that when q2 = 1, r = 2, and when q2 = 2, r = 1, and when q2 = 3, r = 0. As above, the ethylene oxide groups (i.e., the "n7" groups) and propylene oxide groups (i.e., the "p2" groups) can occur in reverse order as well as in a random, sequential, or block arrangement. Examples include a mixture of mono-, di- and tri-(alkyltetraglycolether)-o-phosphoric acid esters generally referred to as trilaureth-4-phosphate commercially available under the trade designation HOSTAPHAT 340KL from Clariant Corp., as well as PPG-5 ceteth 10 phosphate available under the trade designation CRODAPHOS SG from Croda Inc., Parsipanny, NJ, and mixtures thereof.

Surfactants of the amphoteric type include surfactants having tertiary amine groups, which may be protonated, as well as quaternary amine containing zwitterionic surfactants. Examples include:

Ammonium Carboxylate Amphoterics. This class of surfactants can be represented by the following formula:

R²⁹-(C(O)-NH)ₐ-R³⁰-N⁺(R³¹)₂-R³²-COO⁻,

wherein: a = 0 or 1; R²⁹ is a (C₁-C₂₁) alkyl group (saturated or unsaturated straight, branched, or cyclic group), (C₆-C₂₂) aryl group, or (C₆-C₂₂) aralkyl or alkaryl group (saturated straight, branched, or cyclic alkyl group), wherein R²⁹ may be optionally substituted with one or more N, O, or S atoms, or one or more hydroxyl, carboxyl, amide, or amine groups; R³¹ is H or a (C₁-C₈)alkyl group (saturated or unsaturated straight, branched, or cyclic group), wherein R³¹ may be optionally substituted with one or more N, O, or S atoms, or one or more hydroxyl, carboxyl, amine groups, a (C₆-C₉)aryl group, or a (C₆-C₉)aralkyl or alkaryl group; and R³⁰ and R³² are each independently a (C₁-C₁₀) alkylene group that may be the same or different and may be optionally substituted with one or more N, O, or S atoms, or one or more hydroxyl or amine groups.

In the formula above, R²⁹ may be a (C₁-C₁₈) alkyl group, R³¹ may be a (C₁-C₂) alkyl group possibly substituted with a methyl, benzyl group or a methyl group. When R³¹ is H, the surfactant, at higher pH values, could exist as a tertiary amine with a cationic counterion such as Na, K, Li, or a quaternary amine group.

Examples of such amphoteric surfactants include, but are not limited to: certain betaines such as cocobetaine and cocamidopropyl betaine (commercially available under the trade designations MACKAM CB-35 and MACKAM L from McIntyre Group Ltd., University Park, IL); monoacetates such as sodium lauroamphoacetate; diacetates such as disodium lauroamphoacetate; amino- and alkylamino-propionates such as lauraminopropionic acid (commercially available under the trade designations MACKAM 1L, MACKAM 2L, and MACKAM 151L, respectively, from McIntyre Group Ltd.).

Ammonium Sulfonate Amphoterics. This class of amphoteric surfactants is referred to as "sultaines" or "sulfobetaines" and can be represented by the following formula:

R²⁹-(C(O)-NH)ₐ-R³⁰-N⁺(R³¹)₂-R³²-SO₃⁻,

wherein R²⁹-R³² and "a" are defined above. Examples include cocamidopropylhydroxysultaine (commercially available as MACKAM 50-SB from McIntyre Group Ltd.). The sulfoamphoterics may be preferred over the carboxylate amphoterics since the sulfonate group will remain ionized at much lower pH values.

N-acyl amide carboxylate surfactants can be represented by the following formula:

R¹³-C(O)-NR³⁴CH₂-COOM,

wherein: R³³ is a (C₇-C₂₁) alkyl group (saturated or unsaturated straight, branched, or cyclic group), a (C₆-C₂₂) aryl group, or a (C₆-C₂₂) aralkyl or alkaryl group (saturated straight, branched, or cyclic alkyl group), wherein R³³ may be optionally substituted with one or more N, O, or S atoms, or one or more hydroxyl, carboxyl, amide, or amine groups; R³⁴ is H or a (C₁-C₃) alkyl group (saturated straight or branched group). M is defined above. Examples include lauroyl sarcosine, myristoyl sarcosine, oleyl sarcosine, lauroyl glycine, N-Methyl-N-(1-oxododecyl) glycine, and the like. N-acyl sarcosinates are available from Croda Inc. Edison, New Jersey. This class of surfactants is particularly appealing for biodegradable applications since they are readily degraded especially at alkaline pH.

Nonionic surfactants include, but are not limited to, alkyl glucosides, alkyl polyglucosides, polyhydroxy fatty acid amides, sucrose esters, esters of fatty acids and polyhydric alcohols, fatty acid alkanolamides, ethoxylated fatty acids, ethoxylated aliphatic acids, ethoxylated fatty alcohols (e.g., octyl phenoxy polyethoxyethanol available under the trade name TRITON X-100 and nonyl phenoxy poly(ethyleneoxy) ethanol available under the trade name NONIDET P-40, both from Sigma Chemical Company, St. Louis, MO), ethoxylated and/or propoxylated aliphatic alcohols (BRIJ from ICI, Wilmington, DE), ethoxylated glycerides, ethoxylated/propoxylated block copolymers such as PLURONIC and TETRONIC surfactants (BASF), ethoxylated cyclic ether adducts, ethoxylated amide and imidazoline adducts, ethoxylated amine adducts, ethoxylated mercaptan adducts, ethoxylated condensates with alkyl phenols, ethoxylated nitrogen-based hydrophobcs, ethoxylated polyoxypropylenes, polymeric silicones, fluorinated surfactants (FLUORAD-FS 300 surfactant from 3M Company, St. Paul, Minnesota, and ZONYL from Dupont de Nemours Company, Wilmington, Delaware) and polymerizable (reactive) surfactants (e.g., SAM 211 (alkylene polyalkoxy sulfate) surfactant available under the trade name MAZON surfactants useful in the compositions of the present invention are selected from the group consisting of Poloxamers such as PLURONIC from BASF, sorbitan fatty acid esters, and mixtures thereof.

Additionally, the initial compositions may further comprise organic and inorganic fillers. For implantable applications biodegradable, resorbable, or bioerodible inorganic fillers may be particularly appealing. These materials may help to control the degradation rate of the polymer composition. For example, many calcium salts and phosphate salts may be suitable. Exemplary biocompatible resorbable fillers include calcium carbonate, calcium sulfate, calcium phosphate, calcium sodium phosphates, calcium potassium phosphates, tetracalcium phosphate, alpha-tricalcium phosphate, beta-tricalcium phosphate, calcium phosphate apatite, octacalcium phosphate, dicalcium phosphate, calcium carbonate, calcium oxide, calcium hydroxide, calcium sulfate dihydrate, calcium sulfate hemihydrate, calcium fluorite, calcium citrate, magnesium oxide, and magnesium hydroxide. A particularly suitable filler is tribasic calcium phosphate (hydroxy apatite).

The initial composition is mixed and heated to form a melt blended composition. The initial composition is heated to as least the melting temperature of the crystalline or semicrystalline polylactic acid material. In some embodiments, the initial composition further comprises an antimicrobial component and an enhancer. The melting temperature of the crystalline or semicrystalline polylactic acid material is usually in a range of 130°C to 180°C, depending on the crystalline or semicrystalline polylactic acid material selected. The nucleating agent in the melt blended composition may be either dissolved or uniformly dispersed in the single liquid phase.

A variety of melt processing equipment and techniques are known in the art for forming melt blended compositions. The semicrystalline polylactic acid material may be introduced into a hopper and fed into the melt processing equipment. The nonpolymeric aliphatic ester diluent and the nucleating agent can be added to the melt processing equipment through various addition means at the same or different locations on the equipment as that of the semicrystalline polylactic acid material. The individual components (i.e., semicrystalline polylactic acid material, nonpolymeric aliphatic ester diluent, and the nucleating agent) of the initial composition can be introduced into the melt processing equipment to be heated and mixed for a period of time sufficient to form the melt blended composition. The materials can be heated to at least the melting temperature of the semicrystalline polylactic acid material for forming a single liquid phase. Once the single liquid phase has been prepared, the single liquid phase may be cooled to form a composition having two continuous phases. Some examples of melt processing equipment include, but are not limited to, extruders (single and twin screw), compounders, and Banbury mixers. Such equipment and techniques are disclosed, for example, in U.S. Patent No. 3,565,985 (Schrenk et al.), U.S. Patent No. 5,427,842 (Bland et. al.), U.S. Patent Nos. 5,589,122 and 5,599,602 (Leonard), and U.S. Patent No. 5,660,922 (Henidge et al.).

In some embodiments, the components of the initial composition further include the antimicrobial component and the enhancer which can be mixed in and conveyed through an extruder to yield a polymer composition having measurable antimicrobial activity, preferably without polymer degradation or side reactions in the melt. The processing temperature is sufficient to mix the semicrystalline polylactic acid material and antimicrobial component, and allow extruding the composition as a film. Potential degradation reactions include transesterification, hydrolysis, chain scission and radical chain decomposition, and process conditions should minimize such reactions.

The melt blended composition is heated to at least the melting temperature of the crystalline or semicrystalline polylactic acid material in the melt processing equipment. The melting temperature of the crystalline or semicrystalline polylactic acid material is usually in a range of 130°C to 180°C. The processing temperature of the melt blended composition can be at least 130°C, at least 150°C, or at least 160°C. The processing temperature of the melt blended composition may be up to 250°C, up to 240°C, or up to 220°C. The processing temperature of the melt blended composition is often in a range of 130°C to 250°C, in a range of 150°C to 240°C , or in a range of 170°C to 220°C. The melt blended composition can be mixed and heated to form a single liquid phase such that the nucleating agent is dispersed or dissolved in it.

The melt blended composition may be formed into an article upon exiting the melt processing equipment. A film, for example, may be formed as the melt blended composition exits the melt processing equipment described above. The article may also be in the form of a film, a tube, a filament, and other shapes.

During cooling of the melt blended composition, as heat is removed from the single liquid phase, the polylactic acid material begins to crystallize. As the polylactic acid material crystallizes to form a crystalline or semicrystalline polylactic acid material matrix, the nonpolymeric aliphatic ester diluent begins to separate from the polylactic acid material. A first phase comprises a crystalline or semicrystalline polylactic acid material matrix, and a second phase comprises the nonpolymeric aliphatic ester diluent. The melt blended composition may phase separate by a solid-liquid phase separation mechanism, a liquid-liquid phase separation mechanism, or a combination of solid-liquid and liquid-liquid phase separation mechanisms. As the polylactic acid material crystallizes in the cooling melt blended composition, domains of the polylactic acid material form with zones of continuity between the domains. The crystalline or semicrystalline polylactic acid material matrix is generally at least partially surrounded or coated by the nonpolymeric aliphatic ester diluent. There are areas of contact between the crystalline or semicrystalline polylactic acid domains since there is a continuum of polylactic acid material from one domain to the next adjacent polylactic acid material domain in the composition. The composition may be solid and is generally transparent. The crystallization temperature of the polylactic acid material within the melt blended composition is usually in a range of 30°C to 140°C.

A nucleating agent may melt during formation of the melt blended composition. During cooling of the melt blended composition, the nucleating agent may crystallize at a temperature greater than the crystallization temperature of the polylactic acid material. The recrystallized nucleating agent can act as a foreign body for crystallization of the polylactic acid material.

Cooling of the melt blended composition for forming a composition having two continuous phases can occur as it exits the melt processing equipment. Further cooling can occur by casting the melt blended composition onto a cooled surface, such as a patterned drum or a chilled roll. In one embodiment, the cooling may occur through the use of a patterned drum wheel. The cooling temperature of the melt blended composition is at least 40°C or at least 50°C. The cooling temperature of the cast melt blended composition may be up to 120°C, up to 110°C, up to 100°C or up to 95°C, The cooling temperature of the cast melt blended composition may be set in a range of 40°C to 120°C, 40°C to 110°C, 40°C to 100°C, or in a range of 50°C to 95°C. As the cooling of the melt blended composition occurs, phase separation can begin between the nonpolymeric aliphatic ester diluent and the semicrystalline polylactic acid material matrix. The composition is formed.

The extent of which a polymeric material, such as a polylactic acid material in a melt blended composition, crystallizes is a consequence of thermodynamic and kinetic factors. The extent to which a polymeric material crystallizes depends further on whether its polymeric structure is conducive to packing into a crystalline state and on the magnitude of the secondary forces of the polymer chains. Crystallization includes the process of spontaneously organizing polymer chains into an ordered configuration. Packing is facilitated for polymer chains having some structural regularity, compactness, streamlining and some degree of flexibility. Stronger secondary forces contribute to the driving force behind ordering and crystallization of polymer chains.

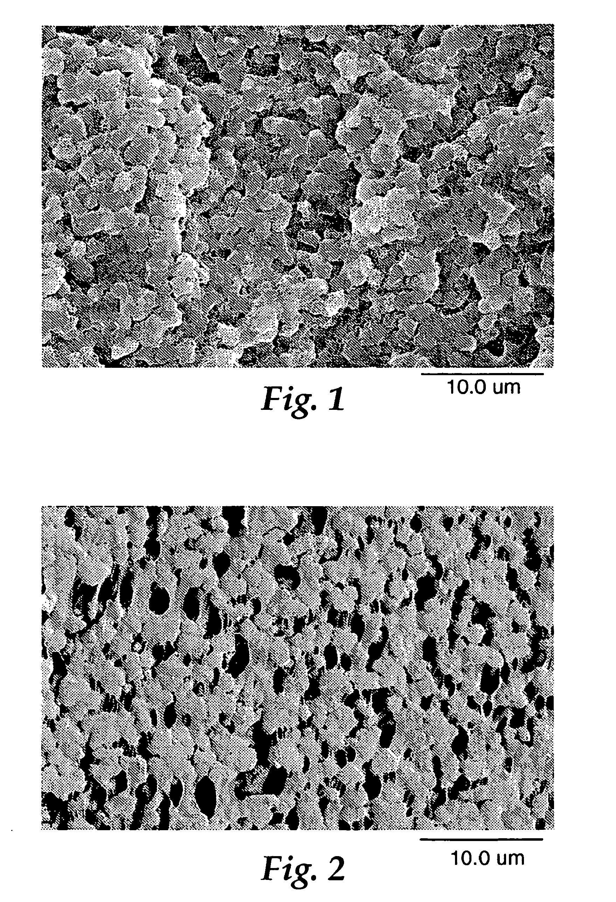

**FIG. 1** illustrates a microporous article having a semicrystalline polylactic acid material matrix of Example 5 before stretching. In **FIG. 1**, at least a portion of the nonpolymeric aliphatic ester diluent has been removed to observe the domains of the semicrystalline polylactic acid material matrix. The formed semicrystalline polylactic acid material matrix shows semicrystalline polylactic acid material domains with minimal separation between adjacent domains.

A network of interconnected pores may be formed by at least partially removing a portion of the nonpolymeric aliphatic ester diluent from the composition having two continuous phases. In some embodiments, the nonpolymeric aliphatic ester diluent may be optionally removed from the composition. Removal may be accomplished by known techniques such as drying and washing. Generally, the microporous article is opaque or semi-transparent after partial removal of the nonpolymeric aliphatic ester diluent.

A network of interconnected micropores may also be formed by stretching the composition, or by a combination of stretching and at least partially removing at least a portion of the nonpolymeric aliphatic ester diluent from the composition. Optionally, the nonpolymeric aliphatic ester diluent may be removed from the composition. The nonpolymeric aliphatic ester diluent that is not removed by washing remains coated on or at least partially surrounds the surfaces of the microporous article. After orienting or stretching the composition, the semicrystalline polylactic acid material domains are pulled apart, permanently attenuating the semicrystalline polylactic acid material in the zones of continuity thereby forming fibrils that interconnect the semicrystalline polylactic acid material domains. Minute voids can be formed between coated semicrystalline polylactic acid material domains, creating a network of interconnected micropores, wherein the microporous articles are generally opaque or semi-transparent

A network of interconnected micropores can be formed by stretching the composition in at least one direction. In one embodiment, the composition may be formed by stretching in two directions. Stretching the composition may be accomplished by pulling the composition with a length orienter, a tenter (i.e., orienting down-web, cross-web, or both), or by other known stretching methods. When the composition is pulled in more than one direction, the degree of stretching may be the same or different in each direction. Stretching of the composition to form a network of interconnected micropores of the microporous article may be in a range of 10 percent to 500 percent of the unstretched area of the composition.

**FIG. 2** illustrates a microporous article after stretching the composition formed in Example 10. The microporous article of **FIG. 2** contains at least a portion of the nonpolymeric aliphatic ester diluent, and is stretched to illustrate the presence of the semicrystalline polylactic acid material domains. The semicrystalline polylactic acid material domains are partially separated from one another to provide for a network of interconnected micropores therebetween. The semicrystalline polylactic acid material domains are connected to each other by fibrils, which radiate from each semicrystalline polylactic acid material domain to adjacent semicrystalline polylactic acid material domains. The semicrystalline polylactic acid material domains are generally spherulitic (i.e., spherical) or may be spherulitic or may be an agglomerate of spherulites. There are areas of contact between adjacent semicrystalline polylactic acid material domains where there is a continuum of polymer from one semicrystalline polylactic acid material domain to the next adjacent semicrystalline polylactic acid material domain in such zones of continuity. The semicrystalline polylactic acid material domains may be at least partially coated by the nonpolymeric aliphatic ester diluent, or optionally the nonpolymeric aliphatic ester diluent may be removed from the semicrystalline polylactic acid material domains. The nonpolymeric aliphatic ester diluent generally occupies at least a portion of the space between the semicrystalline polylactic acid material domains.

In some embodiments, the nonpolymeric aliphatic ester diluent may be optionally removed or at least partially removed from the composition for forming a microporous article. The semicrystalline polylactic acid material domains may not be coated or at least partially coated by the nonpolymeric aliphatic ester diluent.

In some embodiments, the elongation of a microporous article increases with a nucleating agent. The nucleating agent may provide semicrystalline polylactic acid domains having greater elasticity in contrast to a microporous article without a nucleating agent.

In one aspect, a composition having two continuous phases is described. The microporous article comprises a first phase and a second phase. The first phase comprises 40 to 80 weight percent of a crystalline or semicrystalline polylactic acid material, and 0.01 to 10 weight percent of a nucleating agent. The weight percent of each of the crystalline or semicrystalline polylactic acid material and the nucleating agent is based on the total weight of the composition.
The second phase comprises 20 to 60 weight percent of a nonpolymeric aliphatic ester diluent based on the total weight of the composition. The first phase is at least partially surrounded by the second phase in the composition.

In another aspect, a microporous article comprises a crystalline or semicrystalline polylactic acid material, a nucleating agent, and optionally a nonpolymeric aliphatic ester diluent. The microporous article has a network of interconnected micropores therebetween. The microporous article is characterized by a multiplicity of spaced, spherulitic crystalline or semicrystalline polylactic acid material domains. The adjacent domains are connected to each other by a plurality of fibrils comprising polylactic acid material.

Microporous articles of this disclosure may have utility in applications including agricultural, medical hygiene, filtration, barrier, industrial, disposable, and personal care applications. In particular, biodegradable materials can be combined with other materials to enhance strength, flexibility, tensile, degradability, and other related properties. Some example applications of microporous articles include, but are not limited to, diapers, feminine hygiene products, incontinence products, rain wear, surgical gowns, landscaping film, oil clear films (e.g., facial applications), battery separator sheets, and house wrap sheets.

Antimicrobial microporous articles formed from the initial composition having an antimicrobial component and an enhancer may be made by processes known in the art for making products like polymer sheet from polymer resins. For many applications, such microporous articles can be placed in water at 23 °C without substantial loss of physical integrity (e.g. tensile strength) after being immersed 2 hours and dried. Typically, these articles contain little or no water. The water content in the microporous article after extruding, injection molding or solvent casting is typically less than 10% by weight, preferably less than 5% by weight, more preferably less than 1% by weight and most preferably less than 0.2% by weight. Polymeric sheets may be formed by an extrusion process from the initial compositions, resulting in antimicrobial polymer sheets (microporous articles) useful in applications such as food wrapping. Examples of some antimicrobial microporous articles can include sheets, filter membranes, and electrolytic cell membranes. Other articles that may be made of the inventive composition may include medical drapes and gowns, including surgical drapes, procedural drapes, plastic specialty drapes, incise drapes, barrier drapes, barrier gowns, SMS gowns, and the like, wound dressings, wound absorbents, wound contact layers, surgical sponges use to absorb blood and body fluids during surgery, surgical implants, vascular catheters, urinary catheters, endotracheal tubes, shunts, wound drains and other medical devices. Articles made of the initial compositions may be solvent, heat, or ultrasonically welded together as well as being welded to other compatible articles. The initial compositions may be used in conjunction with other materials to form constructions such as sheath/core materials, laminates, compound structures of two or more materials, or useful as coatings on various medical devices. The initial compositions of the present disclosure may be useful in the fabrication of surgical sponges.

The initial compositions are particularly suitable for use in surgical drapes and gowns due to their unique combination of properties. For example, the polylactic acid material/antimicrobial component initial compositions have exceptional antimicrobial activity as described herein. Non-woven web and sheets comprising the initial compositions have good tensile strength; can be heat sealed to form strong bonds allowing specialty drape fabrication; can be made from renewable resources which can be important in disposable products; can have high surface energy to allow wettability and fluid absorbency in the case of non-wovens (contact angles with distilled water often are less than 50 degrees, preferably less than 30 degrees, and most preferably less than 20 degrees when measured on a flat film using the half angle technique described in U. S. Patent No. 5,268,733 and a Tantec Contact Angle Meter, Model CAM-micro, Schaumberg, IL. In order to determine the contact angle of materials other than films a film of the exact same composition should be made by solvent casting the initial composition as described in the examples). It is believed that such webs can be sterilized by gamma radiation or electron beam without significant loss of physical strength (tensile strength for a 1 mil thick film does not decrease by more than 20% and preferably by not more than 10% after exposure to 2.5 Mrad gamma radiation from a cobalt gamma radiation source and aged at 23 ° - 25 °C for 7 days. Additional melt additive (e.g., fluorochemical melt additive) can be added to the initial composition to decrease surface energy (increase the water contact angle) and impart water repellency. When water repellency is desired the contact angle measured on a flat film using the half angle technique as described above is preferably greater than 70 degrees, preferably greater than 80 degrees and most preferably greater than 90 degrees.

The release of an antimicrobial component of the initial composition may improve microporous articles such as wound and surgical dressings by helping to prevent bacterial growth or attachment. The rate of release of antimicrobial components from the semicrystalline polylactic acid material may be affected by incorporation of plasticizers, surfactants, emulsifiers, enhancers, humectants, as well as other components. Suitable humectants may include polyhydric alcohols such as glycerol, propylene glycol, dipropylene glycol, polypropylene glycol, polyethylene glycol, diethylene glycol, pentaerythritol, trimethylolpropane, trimethylolethane, trimethylolbutane, sorbitol, mannitol, xylitol, pantothenol, ethylene glycol adducts of polyhydric alcohol, propylene oxide adducts of polyhydric alcohol, 1,3-butanediol, dipropylene glycol, diglycerine, polyglycerine, erythritol, sorbitan, sugars (e.g., sucrose, glucose, fructose, mannose, xylose, saccharose, trehalose), sugar alcohols, and the like. Potentially useful polyhydric alcohols include glycols (i.e., those containing two hydroxyl groups) including glycerin and propylene glycol.

Other medical devices that may be made, in whole or in part, of the inventive initial composition include: sutures, suture fasteners, surgical mesh, slings, orthopedic pins (including bone filling augmentation material), adhesion barriers, stents, guided tissue repair/regeneration devices, articular cartilage repair devices, nerve guides, tendon repair devices, atrial septal defect repair devices, pericardial patches, bulking and filling agents, vein valves, bone marrow scaffolds, meniscus regeneration devices, ligament and tendon grafts, ocular cell implants, spinal fusion cages, skin substitutes, dural substitutes, bone graft substitutes, bone dowels, and hemostats.

If the initial composition for forming a microporous article is used in a wound dressing backing film, the film may be partially (e.g. zone or pattern) coated or completely coated with various adhesives, including but not limited to pressure sensitive adhesives (PSAs), such as acrylic and block copolymer adhesives, hydrogel adhesives, hydrocolloid adhesives, and foamed adhesives. PSAs can have a relatively high moisture vapor transmission rate to allow for moisture evaporation. Suitable pressure sensitive adhesives include those based on acrylates, polyurethanes, KRATON and other block copolymers, silicones, rubber based adhesives as well as combinations of these adhesives. The preferred PSAs are the normal adhesives that are applied to skin such as the acrylate copolymers described in U.S. Pat. No. RE 24,906, the disclosure of which is hereby incorporated by reference, particularly a 97:3 iso-octyl acrylate:acrylamide copolymer. Also preferred is an 70:15:15 isooctyl acrylate-ethyleneoxide acrylate:acrylic acid terpolymer, as described in U.S. Pat. No. 4,737,410 (Example 31), the disclosure of which is hereby incorporated by reference. Other useful adhesives are described in U.S. Pat. Nos. 3,389,827, 4,112,213, 4,310,509 and 4,323,557, the disclosures of which are hereby incorporated by reference. Inclusion of medicaments or antimicrobial agents in the adhesive is also contemplated, as described in U.S. Pat. Nos. 4,310,509 and 4,323,557.

The disclosure will be further clarified by the following examples which are exemplary and not intended to limit the scope of the disclosure.

### Examples

The present disclosure is more particularly described in the following examples that are intended as illustrations only, since numerous modifications and variations within the scope of the present disclosure will be apparent to those skilled in the art. Unless otherwise noted, all parts, percentages, and ratios reported in the following examples are on a weight basis, and all reagents used in the examples were obtained, or are available, from the chemical suppliers described below, or may be synthesized by conventional techniques.

### Materials

Polylactic Acid (PLA) was obtained from Mitsui Chemical of Tokyo, Japan under the trade designation, LACEA H-400. The PLA (T_{g} ∼ 55 °C; M_{w} ~ 200,000 g/mol) as received was dried for at least 24 hours at 60°C prior to use. PLA was used in Examples 1-20.

Crystalline Polylactic Acid (CPLA; trade designation NatureWorks 4032D) was obtained from NatureWorks LLC; Minneapolis, Minnesota. CPLA was dried in a vacuum oven at 60 °C for 24 hours prior to use, and used in Examples 21-28.

Glycerin Diacetomonolaurate (Diluent I) was obtained from Riken Vitamin Co., LTD of Tokyo, Japan under the trade designation, RIKEMAL PL-012.

Glycerin Diacetomonocaprylate / Glycerin Diacetomonocaprate mixture (Diluent II) was obtained from Riken Vitamin Co., LTD of Tokyo, Japan under the trade designation, RIKEMAL PL-019,used in Examples 21-28.

Glycerin Diacetomonooleate (Diluent III) was obtained from Riken Vitamin Co., LTD of Tokyo, Japan under the trade designation, POEM G-038.

Acetyl Tributyl-Citrate (Diluent IV) was obtained from Asahi Kasei Fine Chemicals Company, Inc. of Osaka, Japan under the trade designation, ATBC, used in Examples 21-28.

Copper Phthalocyanine (Nucleating Agent I) was obtained from Dainichiseika Color & Chemicals Mfg. Co., Inc. of Tokyo, Japan under the trade designation, CHROMOFINE CYANINE GREEN 2GN.

Zinc Phenylphosphonic acid (Nucleating Agent II) was obtained from Nissan Chemical Industries, LTD. of Tokyo, Japan under the trade designation, PPA-ZN, used in Examples 21-28.

Polypropylene (Nucleating Agent III) was obtained from Exxon Mobil Chemicals of Houston, Texas under the trade designation, PP 1024 EA.

Fatty Acid monoester (propylene glycol monolaurate - a mixture of glycerin diaceto monocaprylate and glycerin diaceto moncaprate) was obtained from Riken Vitamin Co., LTD of Tokyo, Japan under the trade designation, RIKEMAL PL-100, used in Examples 21-28.

Enhancer (oligomer of lactic acid and glycolic acid (OLGA)) - Preparatory Example 1. Enhancer was prepared by condensation of lactic acid and glycolic acid. A mixture of L-lactic acid (85-92.0 wt. % in water) 194 grams, 1.9 mol) and glycolic acid (70 wt. % in water, 206 grams, 1.9 mol) was place in a 0.5 liter two-neck flask with stirring under a nitrogen (g) purge. The mixture was raised to a temperature of 185°C for about 14 hours, and then cooled to room temperature. Molecular weight determination results: number average molecular weight (Mn) = 510; g/mole and weight average molecular weight (Mw) = 870g/mole.

### Test Methods and Procedures

### Oil Absorbency (Hydrophobicity)

The microporous articles of this disclosure are generally opaque. Time for absorbency was recorded from the addition of oil to the article to the time where a change in the transparency of the article was observed. A drop of RIKEMAL PL-019 was carefully placed on the surface of the microporous article. The time was recorded when the article turned from opaque to semi-transparent. Oil absorbance or hydrophobicity was characterized as follows: excellent (less than 10 seconds), good (10 to 60 seconds), and poor (greater than 60 seconds).

### Water Absorbency (Hydrophilicity)

The microporous articles of this disclosure are generally opaque. Time for absorbency was recorded from the addition of distilled water to the article to the time where a change in the transparency of the article was observed. A drop of distilled water was carefully placed on the surface of the microporous article. The time was recorded when the article turned from opaque to semi-transparent. Water absorbance or hydrophilicity was characterized as follows: excellent (less than 10 seconds), good (10 to 60 seconds), and poor (greater than 60 seconds).

### Porosity

The porosity of the microporous articles was measured according to JIPS P-8117 (Gurley densometer type B) utilizing a volume of air (100 cm³). The air resistance of the article was measured by recording the time necessary for 100 cm³ of air to pass through an article having an area of 642 mm².

### Cooling

The melt blended composition exited the melt extruder through a slot die to form an article, such as a film, and was positioned onto a cooled casting drum. The film had a thickness in a range of 70 to 140 micrometers prior to stretching. The article was quenched on the cast wheel set at a temperature in a range of 20°C - 120°C. The article was then wound onto a roll.

### Stretching

The microporous articles were stretched in the machine direction and the cross direction. The article was stretched using steel frames, and was stretched in a range of 10 percent to 500 percent. The article was positioned between the steel frames to fix its shape, and then annealed in an oven at a temperature in a range of 50°C - 140°C for at least 0.5 minutes.

### Nonpolymeric Aliphatic Ester Diluent Removal

The nonpolymeric aliphatic ester diluent of the microporous article was removed by solvent extraction. The microporous article was submerged in toluene for about 12 hours to extract at least a portion of the nonpolymeric aliphatic ester diluent. The removal of the nonpolymeric aliphatic ester diluent may occur before stretching or after stretching the microporous article.

### Examples 1 - 20 and Comparative Example 1 (CE 1)

An initial composition comprising the components of a semicrystalline polylactic acid material, a nonpolymeric aliphatic ester diluent, and a nucleating agent material were added to a 20 mm twin screw melt extruder (commercially available from Technovel Corporation of Osaka, Japan under trade designation, KZW20TW-90MG) to heat and mix the components. The semicrystalline polylactic acid material was added through a dry feed hopper at a rate of approximately 2 - 3 kg/hour. The nonpolymeric aliphatic ester diluent was added through a liquid feeding device at a rate of 0.1 to 2 kg/hour. The nucleating agent was added through a solid feeding device at a rate of approximately 0.01 to 0.05 kg/hour. The components were added to the twin screw extruder which was operated at a screw speed of 500 rpm with a first zone temperature of 200°C, and a second zone temperature of 160°C. The twin screw extruder was operated at a screw speed of 125 rpm for Example 20. The melt extruder was equipped with a slot die (commercially available from Research Laboratory Plastics Technology Co., LTD of Osaka, Japan) at the exit location having dimensions of 350 mm x 0.5 mm.

The semicrystalline polylactic acid material, nonpolymeric aliphatic ester diluent and the nucleating agent were mixed and heated above the melting temperature of the semicrystalline polylactic acid material to a temperature of at least 200°C in the first zone of the extruder and mixed further at 160°C in the second zone of the extruder to form the melt blended composition. The slot die at the exit of the melt extruder was set at a temperature in a range of 140°C to 200°C. The melt blended composition was extruded through the slot die for forming an article (e.g., film) having a thickness in a range of 60 micrometers to 150 micrometers. The film was quenched immediately upon contact with a cast wheel having a temperature of 20°C to 95°C. The cooled cast wheel was positioned below the slot die of the twin screw extruder. From the cast wheel, the film was optionally wound at a rate of about 2.0 m/minute onto a roll. In Examples 1-20 and CE 1, the films were either stretched, washed to remove a portion of the nonpolymeric aliphatic ester diluent, or a combination thereof to form a microporous article. Some of the films were stretched in the cross direction (CD) and the machine direction (MD).

Table 1 and Table 2 list the materials for the initial compositions of Examples 1-20 and Comparative Example 1, and their corresponding processing conditions. In Example 7, the film was first stretched, and then a portion of the nonpolymeric aliphatic ester diluent was removed. The film of Example 15 was not stretched, and the nonpolymeric aliphatic ester diluent was removed. The film of Comparative Example 1 (without a nucleating agent) broke during stretching.

**Table 1: Microporous Article Initial Compositions (weight percent (wt. %))**

| **Sample Numbe r** | **PLA (wt. %)** | **Diluent I (wt. %)** | **Diluent II (wt. %)** | **Diluent III (wt. %)** | **Diluent IV (wt. %)** | **Nucleating Agent I (wt. %)** | **Nucleating Agent II (wt. %)** | **Nucleating Agent III (wt. %)** |
|---|---|---|---|---|---|---|---|---|
| 1 | 59.4 | 39.6 | | | | 1.0 | | |
| 2 | 59.4 | 39.6 | | | | | 1.0 | |
| 3 | 59.4 | 39.6 | | | | | 1.0 | |
| 4 | 59.4 | | 39.6 | | | | 1.0 | |
| 5 | 59.4 | | 39.6 | | | | 1.0 | |
| 6 | 59.4 | | 39.6 | | | | 1.0 | |
| 7* | 59.4 | | | 39.6 | | | 1.0 | |
| 8 | 59.4 | | | 39.6 | | | 1.0 | |
| 9* | 60 | | | 40 | | | 1.0 | |
| 10 | 59.4 | | | 39.6 | | | 1.0 | |
| 11 | 64.4 | | 34.6 | | | | 1.0 | |
| 12 | 64.4 | | 34.6 | | | | 1.0 | |
| 13 | 54.5 | | 44.6 | | | | 1.0 | |
| 14 | 59.4 | | | | 39.6 | | 1.0 | |
| 15 | 59.4 | | | | 39.6 | | 1.0 | |
| 16* | 39.6 | | 59.4 | | | | 1.0 | |
| 17 | 59.7 | | 39.8 | | | | 0.5 | |
| 18 | 59.7 | | 39.8 | | | | 0.5 | |
| 19* | 59.4 | | | 39.6 | | | 1.0 | |
| 20 | 54.9 | | 42.5 | | | | | 2.6 |
| CE 1 | 60 | | 40 | | | | | |

| | | | | | | | | |
|---|---|---|---|---|---|---|---|---|
| * Reference Example | | | | | | | | |

**Table 2: Microporous Article Processing Parameters**

| **Sample Number** | **Cooling Temperature (°C) (Cast Wheel)** | **Stretching Temperature (°C)** | **MD x CD (percent)** | **Diluent Removal** |
|---|---|---|---|---|
| 1 | 70 | 20 | 220 x 220 | No |
| 2 | 80 | 60 | 150 x 150 | No |
| 3 | 80 | 60 | 200 x 200 | No |
| 4 | 60 | 60 | 150 x 150 | No |
| 5 | 60 | 60 | 200 x 200 | No |
| 6 | 80 | 60 | 150 x 150 | No |
| 7* | 30 | 60 | 150 x 150 | Yes |
| 8 | 80 | 60 | 150 x 150 | No |
| 9* | 30 | 60 | 150 x 150 | No |
| 10 | 80 | 60 | 200 x 200 | No |
| 11 | 60 | 60 | 150 x 150 | No |
| 12 | 60 | 80 | 180 x 180 | No |
| 13 | 80 | 60 | 140 x 140 | No |
| 14 | 60 | 60 | 150 x 150 | No |
| 15 | 60 | 80 | 180 x 180 | No |
| 16* | 60 | ------- | ------- | Yes |
| 17 | 40 | 60 | 150 x 150 | No |
| 18 | 40 | 60 | 180 x 180 | No |
| 19* | 30 | 60 | 150 x 150 | No |
| 20 | 60 | 60 | 175 x 1 | No |
| CE 1 | 60 | 60 | 150 x 150 (film break) | No |

| | | | | |
|---|---|---|---|---|
| * Reference Example | | | | |

Table 3 lists the thicknesses of the microporous articles, occurrence of phase separation, water and oil absorbency results, and porosity (Gurley) testing results. Hydrophobic / hydrophilic properties of Examples 1-20 and Comparative Example 1 are shown in Table 3. Phase separation of the films was determined by an opaque or semi-transparent film. Oil and water absorbance ratings are listed in Table 3.

**Table 3: Microporous Article Properties**

| **Sample Number** | **Thickness (micrometers )** | **Phase Separation (Yes/No)** | **Oil Absorbance** | **Water Absorbance** | **Porosity (Gurley) (seconds)** |
|---|---|---|---|---|---|
| 1 | 45 | Yes | Good | Poor | 6000 |
| 2 | 65 | Yes | Good | Poor | 550 |
| 3 | 35 | Yes | Good | Poor | 190 |
| 4 | 80 | Yes | Excellent | Excellent | 51 |
| 5 | 60 | Yes | Excellent | Poor | 27 |
| 6 | 80 | Yes | Good | Poor | 141 |
| 7* | 65 | Yes | Excellent | Poor | 730 |
| 8 | 65 | Yes | Good | Poor | 3100 |
| 9* | 65 | No | Poor | Not analyzed | none |
| 10 | 45 | Yes | Excellent | Poor | 1100 |
| 11 | 65 | Yes | Excellent | Poor | 1200 |
| 12 | 60 | Yes | Excellent | Poor | 340 |
| 13 | 65 | Yes | Excellent | Poor | 330 |
| 14 | 75 | Yes | Excellent | Poor | 230 |
| 15 | 55 | Yes | Excellent | Poor | 36 |
| 16* | 90 | Yes | Excellent | Poor | 650 |
| 17 | 55 | Yes | Excellent | Excellent | 130 |
| 18 | 45 | Yes | Excellent | Good | 63 |
| 19* | 65 | No | Poor | Not analyzed | none |
| 20 | 100 | Yes | Good | Poor | 230 |
| CE 1 | Not analyzed | Not analyzed | Not analyzed | Not analyzed | Not analyzed |

| | | | | | |
|---|---|---|---|---|---|
| * Reference Examples | | | | | |

### Examples 21 - 28 and Comparative Example 2 (CE 2)

An initial composition comprising the components of a Semicrystalline polylactic acid material, a nonpolymeric aliphatic ester diluent (ATBC), a nucleating agent, an antimicrobial component, and an enhancer were added to a 20 mm twin screw melt extruder (commercially available from Technovel Corporation of Osaka, Japan under trade designation, KZW20TW-90MG) to heat and mix the components. The semicrystalline polylactic acid material was added through a dry feed hopper at a rate of approximately 2 - 3 kg/hour. The nonpolymeric aliphatic ester diluent was added through a liquid feeding device at a rate of 0.1 to 2 kg/hour. The nucleating agent (PPA-Zn) was added through a solid feeding device at a rate of approximately 0.01 to 0.05 kg/hour. The antimicrobial component and the enhancer independently were added through a liquid feeding device at a rate of 0.01 to 2 kg/hour. The components were added to the twin screw extruder which was operated at a screw speed of 500 rpm with a first zone temperature of 200°C, and a second zone temperature of 160°C for Examples 21-28. The melt extruder was equipped with a slot die (commercially available from Research Laboratory Plastics Technology Co., LTD of Osaka, Japan) at the exit location having dimensions of 350 mm x 0.5 mm. The slot die at the exit of the melt extruder was set at a temperature of 160°C. The melt blended composition was extruded through the slot die for forming an article (e.g., film) having a thickness in a range of 45 micrometers to 150 micrometers. The film was quenched immediately upon contact with a cast wheel having a temperature of 60°C to 95°C. The cooled cast wheel was positioned below the slot die of the twin screw extruder. From the cast wheel, the film was optionally wound at a rate of about 2.0 m/minute onto a roll. For Examples 22-23, and 25-28, the films were stretched in the machine-direction and the transverse(cross)-direction (CD) simultaneously in a batch stretcher. Stretching conditions (e.g., stretching temperature, stretching ratio for both directions) are shown in Table 5. The stretched article was placed between two steel frames to set (fix) the shape, and then the shape was annealed in a convection oven at a temperature similar to the stretching temperature for about 1 minute.

Table 4 lists the materials for the initial compositions of Examples 21-28, and Table 5 lists their corresponding processing conditions. Comparative Example 2 (CE 2) is a polyester terephthalate film (PET; thickness-100 micrometers) (Eastman Chemical, Kingsport, Tennessee).

**Table 4: Antimicrobial Microporous Article Initial Compositions**

| **Sample Number** | **PLA (wt. %)** | **Diluent (wt. %)** | **Antimicrobial Component (wt. %)** | **Enhancer (wt. %)** | **Nucleating Agent (wt. %)** |
|---|---|---|---|---|---|
| 21 | 62.5 | 28.125 | 4.6875 | 4.6875 | 1 |
| 22 | 62.5 | 28.125 | 4.6875 | 4.6875 | 1 |
| 23 | 62.5 | 28.125 | 4.6875 | 4.6875 | 1 |
| 24 | 65 | 26.25 | 3.75 | 3.75 | 1 |
| 25 | 65 | 26.25 | 3.75 | 3.75 | 1 |
| 26 | 65 | 26.25 | 3.75 | 3.75 | 1 |
| 27 | 65 | 26.25 | 3.75 | 3.75 | 1 |
| 28 | 65 | 26.25 | 3.75 | 3.75 | 1 |

**Table 5: Antimicrobial Microporous Article Processing Parameters**

| **Sample Number** | **Cooling Temperature (°C) (Cast Wheel)** | **Stretching Temperature (°C)** | **MD x CD (percent)** |
|---|---|---|---|
| 21 | 75 | -- | ---- |
| 22 | 75 | 70 | 150 x 150 |
| 23 | 85 | 70 | 150 x 150 |
| 24 | 60 | -- | ---- |
| 25 | 60 | 70 | 150 x 150 |
| 26 | 60 | 70 | 180 x 180 |
| 27 | 60 | 70 | 200 x 200 |
| 28 | 70 | 70 | 150 x 150 |

Table 6 lists the thicknesses of the antimicrobial microporous articles, occurrence of phase separation, oil absorbency results, and porosity (Gurley) testing results. Phase separation of the films was determined by whether the film was opaque or semi-transparent. Oil absorbance ratings are listed in Table 6.

**Table 6: Antimicrobial Microporous Article Properties**

| **Sample Number** | **Thickness (micrometers )** | **Phase Separation (Yes/No)** | **Oil Absorbance** | **Porosity (Gurley) (seconds)** |
|---|---|---|---|---|
| 21 | 98 | Yes | -- | -- |
| 22 | 91 | Yes | Excellent | 173 |
| 23 | 81 | Yes | Good | 3035 |
| 24 | 87 | Yes | -- | -- |
| 25 | 80 | Yes | Excellent | 145 |
| 26 | 50 | Yes | Excellent | 25 |
| 27 | 55 | Yes | Excellent | 27 |
| 28 | 72 | Yes | Excellent | 183 |
| CE 2 | Not analyzed | Not analyzed | Not analyzed | Not analyzed |

Tables 7 and 8 list the results of microbial testing for CE 2 and the antimicrobial microporous articles of Examples 21-28. Examples 21-28 and CE 2 were tested for microbial activity according to Japanese Industrial Standard test number JISZ 2801:2000 ((Antimicrobial Products - Test for Antimicrobial Activity and Efficacy) - English edition published 2001-08). Gram-positive bacteria (Staphylococcus aureus ATCC #6538; ATCC, Washington, DC) and Gram-negative bacteria (Pseudomonas aeruginosa ATCC #9027; ATCC, Washington, DC) were introduced to Examples 21-28 and CE2. Examples 21-28 illustrated in Tables 7 and 8 show antimicrobial activity and efficacy..

**Table 7: Antimicrobial Microporous Article Testing using Staphylococcus aureus**

| **Sample Number** | **CFU/ml** | **SD CFU/ml** | **CFU/sample** | **SD CFU/sample** | **Antimicrobial Activity (R)*** |
|---|---|---|---|---|---|
| t=0 | 69000 | -- | 690000 | --- | --- |
| 21 | <10 | 0 | < 100 | 0 | 4.0 |
| 22 | <10 | 0 | < 100 | 0 | 4.0 |
| 23 | 10 | 7 | 100 | 70 | 4.0 |
| 24 | <10 | 0 | < 100 | 0 | 4.0 |
| 25 | 25 | 35 | 250 | 354 | 3.6 |
| 28 | < 10 | 0 | < 100 | 0 | 4.0 |
| CE 2 | 91500 | 65761 | 915000 | 657609 | --- |

| | | | | | |
|---|---|---|---|---|---|
| * Log reduction vs. t = 0 | | | | | |

**Table 8: Antimicrobial Microporous Article Testing using Pseudomonas aeruginosa**

| **Sample Number** | **CFU/ml** | **SD CFU/ml** | **CFU/sample** | **SD CFU/sample** | **Antimicrobial Activity (R)*** |
|---|---|---|---|---|---|
| t = 0 | 73000 | --- | 730000 | --- | --- |
| 21 | < 10 | 0 | < 100 | 0 | 5.5 |
| 22 | < 10 | 0 | < 100 | 0 | 5.5 |
| 23 | < 10 | 0 | < 100 | 0 | 5.5 |
| 24 | < 10 | 0 | < 100 | 0 | 5.5 |
| 25 | < 10 | 0 | < 100 | 0 | 5.5 |
| 28 | < 10 | 0 | < 100 | 0 | 5.5 |
| CE 2 | 3400000 | 1272792 | 34000000 | 12727922 | --- |

| | | | | | |
|---|---|---|---|---|---|
| * Log reduction vs. t = 0 | | | | | |

## Claims

1. A method for forming a microporous article, the method comprising:
(i) preparing an initial composition comprising
(a) a crystalline or semicrystalline polylactic acid material;
(b) a nonpolymeric aliphatic ester diluent; and
(c) a nucleating agent;
(ii) heating the initial composition to at least a melting temperature of the crystalline or semicrystalline polylactic acid material to form a melt blended composition, wherein the crystalline or semicrystalline polylactic acid material and the nonpolymeric aliphatic ester diluent form a single liquid phase, and the nucleating agent is uniformly dispersed or dissolved in the single liquid phase;
(iii) cooling the melt blended composition to a temperature sufficient for the melt blended composition to phase separate the melt blended composition into a composition having two continuous phases comprising (a) a first phase comprising a crystalline or semicrystalline polylactic acid material matrix and the nucleating agent dispersed throughout the crystalline or semicrystalline polylactic acid material matrix, and (b) a second phase comprising the nonpolymeric aliphatic ester diluent, wherein the cooling temperature of the melt blended composition is at least 40°C; and
(iv) forming a network of interconnected micropores by stretching the composition in at least one direction.

2. The method of claim 1, wherein the nonpolymeric aliphatic ester diluent is of the formula: 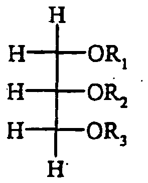 wherein
R₁ comprises an acyl having 6 to 24 carbon atoms;
R₂ comprises hydrogen, or an acyl having 2 to 6 carbon atoms; and
R₃ comprises hydrogen, or an acyl having 2 to 6 carbon atoms.

3. The method of claim 1, wherein the nonpolymeric aliphatic ester diluent is of the formula: 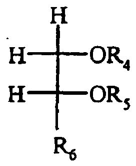 wherein
R₄ comprises an acyl having 6 to 24 carbon atoms;
R₅ comprises hydrogen, or an acyl having 2 to 6 carbon atoms; and
R₆ comprises an alkyl having 1 to 4 carbon atoms.

4. The method of claim 1, wherein the nonpolymeric aliphatic ester diluent is of the formula: 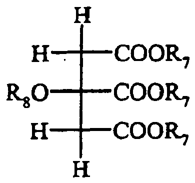 wherein
R₇ independently comprises hydrogen, or an alkyl having 1 to 8 carbon atoms; and
R₈ comprises an acyl having 2 to 5 carbon atoms.

5. The method of any one of claims 1 to 4, wherein the nucleating agent comprises copper phthalocyanine, zinc phenylphosphonate, talc, clay, mica, stereocomplex of poly L-lactic acid and poly D-lactic acid, isotactic polypropylene, or combinations thereof.

6. The method of claim 1, wherein the initial composition further comprises an antimicrobial component.

7. The method of claim 6, wherein the initial composition further comprises an enhancer.

8. A composition obtainable by the method steps (i) to (iii) of claim 1, having two continuous phases comprising:
(a) a first phase comprising 40 to 80 weight percent of a crystalline or semicrystalline polylactic acid material, and 0.01 to 10 weight percent of a nucleating agent, the weight percent of the crystalline or semicrystalline polylactic acid material and the weight percent of the nucleating agent independently being based on the total weight of the composition; and
(b) a second phase comprising 20 to 60 weight percent of a nonpolymeric aliphatic ester diluent based on the total weight of the composition;
wherein the first phase is at least partially surrounded by the second phase.

9. A microporous article obtainable by the method of claim 1, comprising a crystalline or semicrystalline polylactic acid material, a nucleating agent and a nonpolymeric aliphatic ester diluent, the microporous article having a network of interconnected micropores therebetween, the microporous article **characterized by** a multiplicity of spaced, spherulitic crystalline or semicrystalline polylactic acid material domains, adjacent crystalline or semicrystalline polylactic acid material domains being connected to each other by a plurality of fibrils comprising polylactic acid material.

10. An antimicrobial microporous article obtainable by the method of claim 1, comprising a crystalline or semicrystalline polylactic acid material, a nucleating agent, an antimicrobial component, and a nonpolymeric aliphatic ester diluent, the antimicrobial microporous article having a network of interconnected micropores therebetween, the antimicrobial microporous article **characterized by** a multiplicity of spaced, spherulitic crystalline or semicrystalline polylactic acid material domains, adjacent crystalline or semicrystalline polylactic acid material domains being connected to each other by a plurality of fibrils comprising polylactic acid material..

## Patentansprüche

1. Verfahren zur Herstellung eines mikroporösen Artikels, umfassend:
(i) Herstellen einer Ausgangszusammensetzung, die Folgendes umfasst:
(a) ein kristallines oder teilkristallines Polymilchsäurematerial;
(b) ein nichtpolymeres aliphatisches Esterverdünnungsmittel; und
(c) ein Nukleierungsmittel;
(ii) Erhitzen der Ausgangszusammensetzung auf mindestens eine Schmelztemperatur des kristallinen oder teilkristallinen Polymilchsäurematerials, wobei man eine in der Schmelze gemischte Zusammensetzung erhält, in der das kristalline oder teilkristalline Polymilchsäurematerial und das nichtpolymere aliphatische Esterverdünnungsmittel eine einzige flüssige Phase bilden und das Nukleierungsmittel in der einzigen flüssigen Phase einheitlich dispergiert oder gelöst ist;
(iii) Abkühlen der in der Schmelze gemischten Zusammensetzung auf eine Temperatur ausreichend zur Phasentrennung der in der Schmelze gemischten Zusammensetzung zu einer Zusammensetzung mit zwei kontinuierlichen Phasen, umfassend (a) eine erste Phase, die eine Matrix aus kristallinem oder teilkristallinem Polymilchsäurematerial und das in der gesamten Matrix aus kristallinem oder teilkristallinem Polymilchsäurematerial dispergierte Nukleierungsmittel umfasst, und (b) eine zweite Phase, die das nichtpolymere aliphatische Esterverdünnungsmittel umfasst, wobei die Abkühlungstemperatur der in der Schmelze gemischten Zusammensetzung mindestens 40° C beträgt; und
(iv) Bilden eines Netzwerks von miteinander verbundenen Mikroporen durch Strecken der Zusammensetzung in mindestens einer Richtung.

2. Verfahren nach Anspruch 1, bei dem das nichtpolymere aliphatische Esterverdünnungsmittel die folgende Formel aufweist: 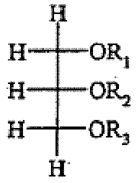 worin
R₁ ein Acyl mit 6 bis 24 Kohlenstoffatomen umfasst;
R₂ Wasserstoff oder ein Acyl mit 2 bis 6 Kohlenstoffatomen umfasst; und
R₃ Wasserstoff oder ein Acyl mit 2 bis 6 Kohlenstoffatomen umfasst.

3. Verfahren nach Anspruch 1, bei dem das nichtpolymere aliphatische Esterverdünnungsmittel die folgende Formel aufweist: 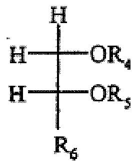 worin
R₄ ein Acyl mit 6 bis 24 Kohlenstoffatomen umfasst;
R₅ Wasserstoff oder ein Acyl mit 2 bis 6 Kohlenstoffatomen umfasst; und
R₆ ein Alkyl mit 1 bis 4 Kohlenstoffatomen umfasst.

4. Verfahren nach Anspruch 1, bei dem das nichtpolymere aliphatische Esterverdünnungsmittel die folgende Formel aufweist: 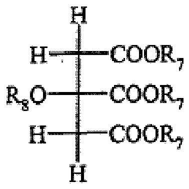 worin
R₇ unabhängig voneinander Wasserstoff oder ein Alkyl mit 1 bis 8 Kohlenstoffatomen umfasst; und
R₈ ein Acyl mit 2 bis 5 Kohlenstoffatomen umfasst.

5. Verfahren nach einem der Ansprüche 1 bis 4, bei dem das Nukleierungsmittel Kupferphthalocyanin, Zinkphenylphosphonat, Talk, Ton, Glimmer, Stereokomplex von Poly-L-milchsäure und Poly-D-milchsäure, isotaktisches Polypropylen oder Kombinationen davon umfasst.

6. Verfahren nach Anspruch 1, bei dem die Ausgangszusammensetzung ferner eine antimikrobielle Komponente umfasst.

7. Verfahren nach Anspruch 6, bei dem die Ausgangszusammensetzung ferner einen Verstärker umfasst.

8. Zusammensetzung, die nach den Verfahrensschritten (i) bis (iii) von Anspruch 1 erhältlich ist und zwei kontinuierliche Phasen aufweist, umfassend:
(a) eine erste Phase, die 40 bis 80 Gewichtsprozent eines kristallinen oder teilkristallinen Polymilchsäurematerials und 0,01 bis 10 Gewichtsprozent eines Nukleierungsmittels umfasst, wobei sich der Gewichtsprozentanteil des kristallinen oder teilkristallinen Polymilchsäurematerials und der Gewichtsprozentanteil des Nukleierungsmittels unabhängig voneinander auf das Gesamtgewicht der Zusammensetzung beziehen; und
(b) eine zweite Phase, die 20 bis 60 Gewichtsprozent eines nichtpolymeren aliphatischen Esterverdünnungsmittels, bezogen auf das Gesamtgewicht der Zusammensetzung, umfasst;
wobei die erste Phase zumindest teilweise von der zweiten Phase umgeben ist.

9. Mikroporöser Artikel, der nach dem Verfahren von Anspruch 1 erhältlich ist und ein kristallines oder teilkristallines Polymilchsäurematerial, ein Nukleierungsmittel und ein nichtpolymeres aliphatisches Esterverdünnungsmittel umfasst, wobei der mikroporöse Artikel ein Netzwerk von miteinander verbundenen Mikroporen dazwischen aufweist, wobei der mikroporöse Artikel durch eine Vielzahl von beabstandeten, sphärulitischen Domänen von kristallinem oder teilkristallinem Polymilchsäurematerial gekennzeichnet ist, wobei benachbarte Domänen von kristallinem oder teilkristallinem Polymilchsäurematerial durch eine Vielzahl von Polymilchsäurematerial umfassenden Fibrillen miteinander verbunden sind.

10. Antimikrobieller mikroporöser Artikel, der nach dem Verfahren von Anspruch 1 erhältlich ist und ein kristallines oder teilkristallines Polymilchsäurematerial, ein Nukleierungsmittel und ein nichtpolymeres aliphatisches Esterverdünnungsmittel umfasst, wobei der antimikrobielle mikroporöse Artikel ein Netzwerk von miteinander verbundenen Mikroporen dazwischen aufweist, wobei der antimikrobielle mikroporöse Artikel durch eine Vielzahl von beabstandeten, sphärulitischen Domänen von kristallinem oder teilkristallinem Polymilchsäurematerial gekennzeichnet ist, wobei benachbarte Domänen von kristallinem oder teilkristallinem Polymilchsäurematerial durch eine Vielzahl von Polymilchsäurematerial umfassenden Fibrillen miteinander verbunden sind.

## Revendications

1. Procédé de formation d'un article microporeux, le procédé consistant à :
(i) préparer une composition initiale comprenant
(a) un matériau acide polylactique cristallin ou semi-cristallin ;
(b) un diluant ester aliphatique non polymère ; et
(c) un agent de nucléation ;
(ii) chauffer la composition initiale au moins jusqu'à une température de fusion du matériau acide polylactique cristallin ou semi-cristallin pour former une composition mélangée par fusion, le matériau acide polylactique cristallin ou semi-cristallin et le diluant ester aliphatique non polymère formant une phase liquide unique, et l'agent de nucléation étant dispersé uniformément ou dissous dans la phase liquide unique ;
(iii) refroidir la composition mélangée par fusion jusqu'à une température suffisante pour que la composition mélangée par fusion subisse une séparation de phase de la composition mélangée par fusion produisant une composition comportant deux phases continues comprenant (a) une première phase comprenant une matrice de matériau acide polylactique cristallin ou semi-cristallin et l'agent de nucléation dispersé à travers la matrice de matériau acide polylactique cristallin ou semi-cristallin, et (b) une deuxième phase comprenant le diluant ester aliphatique non polymère, la température de refroidissement de la composition mélangée par fusion étant d'au moins 40 °C ; et
(iv) former un réseau de micropores interconnectés par étirage de la composition dans au moins une direction.

2. Procédé selon la revendication 1, dans lequel le diluant ester aliphatique non polymère a la formule : 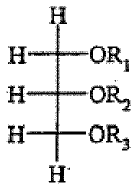 dans laquelle
R₁ comprend un groupe acyle contenant 6 à 24 atomes de carbone ;
R₂ comprend l'hydrogène, ou un groupe acyle contenant 2 à 6 atomes de carbone ; et
R₃ comprend l'hydrogène, ou un groupe acyle contenant 2 à 6 atomes de carbone.

3. Procédé selon la revendication 1, dans lequel le diluant ester aliphatique non polymère a la formule : 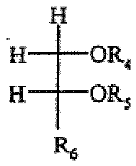 dans laquelle
R₄ comprend un groupe acyle contenant 6 à 24 atomes de carbone ;
R₅ comprend l'hydrogène, ou un groupe acyle contenant 2 à 6 atomes de carbone ; et
R₆ comprend un groupe alkyle contenant 1 à 4 atomes de carbone.

4. Procédé selon la revendication 1, dans lequel le diluant ester aliphatique non polymère a la formule : 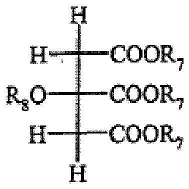 dans laquelle
R₇ comprend indépendamment l'hydrogène, ou un groupe alkyle contenant 1 à 8 atomes de carbone ; et
R₈ comprend un groupe acyle contenant 2 à 5 atomes de carbone.

5. Procédé selon l'une quelconque des revendications 1 à 4, dans lequel l'agent de nucléation comprend la phtalocyanine de cuivre, le phénylphosphonate de zinc, le talc, l'argile, le mica, un stéréocomplexe d'acide poly-L-lactique et d'acide poly-D-lactique, un polypropylène isotactique, ou des combinaisons de ceux-ci.

6. Procédé selon la revendication 1, dans lequel la composition initiale comprend en outre un constituant antimicrobien.

7. Procédé selon la revendication 6, dans lequel la composition initiale comprend en outre un agent de renforcement.

8. Composition pouvant être obtenue par les étapes de procédé (i) à (iii) selon la revendication 1, comportant deux phases continues comprenant :
(a) une première phase comprenant 40 % à 80 % en poids d'un matériau acide polylactique cristallin ou semi-cristallin, et 0,01 % à 10 % en poids d'un agent de nucléation, le pourcentage pondéral du matériau acide polylactique cristallin ou semi-cristallin et le pourcentage pondéral de l'agent de nucléation étant exprimés indépendamment relativement au poids total de la composition ; et
(b) une deuxième phase comprenant 20 % à 60 % en poids d'un diluant ester aliphatique non polymère relativement au poids total de la composition ;
la première phase étant au moins en partie entourée par la deuxième phase.

9. Article microporeux pouvant être obtenu par le procédé selon la revendication 1, comprenant un matériau acide polylactique cristallin ou semi-cristallin, un agent de nucléation et un diluant ester aliphatique non polymère, l'article microporeux comportant un réseau de micropores interconnectés entre ceux-ci, l'article microporeux étant **caractérisé par** une pluralité de domaines espacés, sphérulitiques, de matériau acide polylactique cristallin ou semi-cristallin, des domaines adjacents de matériau acide polylactique cristallin ou semi-cristallin étant reliés les uns aux autres par une pluralité de fibrilles comprenant un matériau acide polylactique.

10. Article microporeux antimicrobien pouvant être obtenu par le procédé selon la revendication 1, comprenant un matériau acide polylactique cristallin ou semi-cristallin, un agent de nucléation, un constituant antimicrobien, et un diluant ester aliphatique non polymère, l'article microporeux antimicrobien comportant un réseau de micropores interconnectés entre ceux-ci, l'article microporeux antimicrobien étant **caractérisé par** une pluralité de domaines espacés, sphérulitiques, de matériau acide polylactique cristallin ou semi-cristallin, des domaines adjacents de matériau acide polylactique cristallin ou semi-cristallin étant reliés les uns aux autres par une pluralité de fibrilles comprenant un matériau acide polylactique.
